(19) Europäisches Patentamt
European Patent Office
Office européen des brevets

(11) Publication number: 0 062 876
B1

(12) **EUROPEAN PATENT SPECIFICATION**

(45) Date of publication of patent specification: 24.09.86

(21) Application number: 82102908.9

(22) Date of filing: 05.04.82

(51) Int. Cl.[4]: C 07 D 205/08, C 07 D 401/12, C 07 D 401/14, C 07 D 403/12, C 07 D 403/14, C 07 D 405/14, C 07 D 417/12, C 07 D 417/14, C 07 D 413/12, C 07 D 413/14

# (54) 2-Oxo-1-(((substituted sulfonyl)amino)carbonyl)azetidines.

(30) Priority: 09.04.81 US 252672

(43) Date of publication of application: 20.10.82 Bulletin 82/42

(45) Publication of the grant of the patent: 24.09.86 Bulletin 86/39

(84) Designated Contracting States: AT BE CH DE FR GB IT LI LU NL SE

(56) References cited:
EP-A-0 001 715
EP-A-0 021 678
BE-A- 849 945

THE JOURNAL OF ORGANIC CHEMISTRY, vol. 40, no. 16, 8th August 1975, pages 2356-9; D.H. AUE et al.: "Addition of p-toluenesulfonyl isocyanate to imino ethers. Isolation of stable 1,4-dipolar intermediate".

Burger's Medicinal Chemistry II (1979), p. 156-157

(73) Proprietor: E.R. Squibb & Sons, Inc.
Lawrenceville-Princeton Road
Princeton, N.J. 08540 (US)

(72) Inventor: Breuer, Hermann
Sauerzapfstrasse 5
D-8411 Schoenhofen (DE)
Inventor: Slusarchiyk, William A.
Sunset Road
Belle Mead New Jersey (US)
Inventor: Denzel, Theodor
Lessingstrasse 12
D-8400 Regensburg (DE)
Inventor: Treuner, Uwe D.
Muellerstrasse 5
D-8400 Regensburg (DE)

(74) Representative: Vossius & Partner
Siebertstrasse 4 P.O. Box 86 07 67
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 062 876 B1

**Description**

Sulfonylamino carbonyl-azetidinones are known from J. Org. Chem. *40,* 1975, pp. 2356—2359. Further, β-lactam compounds having an antibacterial activity are described in EP—A—1715, EP—A—21678, BE—A—849 445 and Burger's Medical Chemistry, Part II (1979), pages 156, 157.

This invention is directed to a novel family of β-lactam antibiotics, and to the use of such compounds as antibacterial agents. It has been discovered that the β-lactam nucleus can be biologically activated by a substituent having the formula

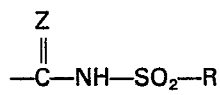

attached to the nitrogen atom in the nucleus.

β-Lactams having a

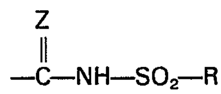

substituent in the 1-position and an acylamino substituent in the 3-position (and salts thereof) exhibit activity against a range of gram-negative and gram-positive bacteria.

The novel family of β-lactam antibiotics of this invention is encompassed by the formula (I)

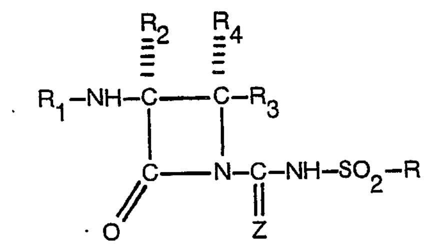

I

and salts thereof.

β-Lactams having the formula (II) are preferred

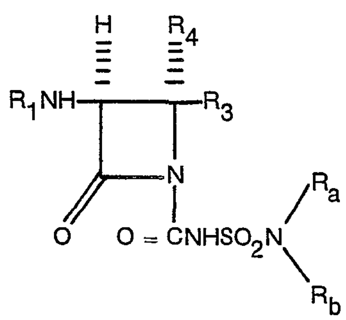

II

In addition to the above described β-lactams having a

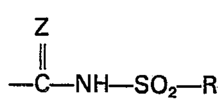

substituent in the 1-position and an acylamino substituent in the 3-position, this invention also encompasses β-lactams having a

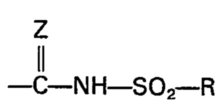

substituent in the 1-position and an amino substituent in the 3-position.

Illustrative compounds of this type have the formula (III)

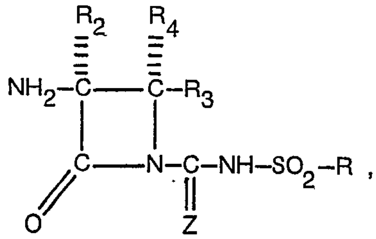

III

and salts thereof.

These compounds are intermediates useful for the preparation of corresponding 3-acylamino compounds.

As used in formulas (I) and (III), and throughout the specification, the symbols are as defined below.

R is alkyl, alkenyl, alkynyl, substituted alkyl, phenyl, substituted phenyl, het which is a 5,6 or 7-membered aromatic or fully or partially saturated heterocycle containing one or more nitrogen, oxygen or sulfur atoms optionally substituted with one or more halogen, hydroxyl, nitro, amino, cyano, trifluoromethyl, $C_1$—$C_4$-alkyl, $C_1$—$C_4$-alkoxy, alkylsulfonyl, phenyl, substituted phenyl, 2-furylimino, benzylimino or substituted $C_1$—$C_4$ alkyl groups, phenylalkyl, (substituted-phenyl)alkyl, het-alkyl or —$NR_aR_b$ wherein $R_a$ and $R_b$ are the same or different and each is hydrogen, alkyl, substituted alkyl, phenyl, substituted phenyl, phenylalkyl, (substituted phenyl)alkyl or —$NR_aR_b$ in which one of $R_a$ and $R_b$ is hydrogen, alkyl, phenyl, substituted phenyl, phenylalkyl or (substituted phenyl)alkyl and the other is amino, alkanoylamino, arylcarbonylamino, alkoxycarbonylamino, alkylsulfonylamino, alkylamino, dialkylamino, phenylamino, (substituted phenyl)amino, hydroxy, cyano, alkoxy, phenyloxy, (substituted phenyl)oxy, phenylalkoxy, (substituted phenyl)alkoxy, het as defined above, het-alkyl, het-alkoxy, alkylsulfonyl, alkylmethyleneamino, phenylmethyleneamino or (substituted phenyl)methyleneamino.

$R_1$ is acyl derived from a carboxylic acid;

$R_2$ is hydrogen or methoxy;

$R_3$ and $R_4$ are the same or different and each is hydrogen, alkyl, cycloalkyl, phenyl or substituted phenyl, or one of $R_3$ and $R_4$ is hydrogen and the other is alkoxycarbonyl, alken-1-yl, alkyn-1-yl, 2-phenylethenyl, 2-phenylethynyl, carboxyl, —$CH_2X_1$ (wherein $X_1$ is azido, amino (—$NH_2$), hydroxy, alkanoylamino, alkylsulfonyloxy, phenylsulfonyloxy, (substituted phenyl)sulfonyloxy, phenyl, substituted phenyl, halogen, benzylthio, (substituted phenyl)methylthio, triphenylmethylthio, cyano or mercapto), —S—$X_2$ or —O—$X_2$ (wherein $X_2$ is alkyl, phenyl, substituted phenyl, alkanoyl, phenylcarbonyl, (substituted phenyl)carbonyl, or heteroarylcarbonyl), or

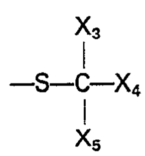 or 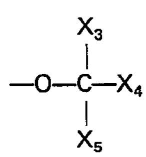

(wherein one of $X_3$ and $X_4$ is hydrogen and the other is hydrogen or alkyl, or $X_3$ and $X_4$ when taken together with the carbon atom to which they are attached form a cycloalkyl group, and $X_5$ is formyl, alkanoyl, phenylcarbonyl, (substituted phenyl)carbonyl, phenylalkylcarbonyl, (substituted phenyl)alkylcarbonyl, carboxyl, alkoxycarbonyl, aminocarbonyl, (substituted amino)carbonyl, or cyano; and

Z is oxygen or sulfur.

The following definitions are to be applied (unless they are otherwise limited in specific instances) either individually or in part of a larger group; these definitions are valid throughout the specification;

alkyl and alkoxy groups contain 1 to 10 carbon atoms, cycloalkyl groups contain 3 to 7 carbon atoms, alkanoyl, alkenyl, alkynyl, alken-1-yl and alkyn-1-yl groups contain 2 to 10 carbon atoms,

substituted phenyl refers to a phenyl group substituted with 1, 2 or 3 amino, halogen, hydroxyl, trifluoromethyl, $C_1$—$C_4$ alkyl, $C_1$—$C_4$ alkoxy or carboxyl,

substituted alkyl refers to $C_1$—$C_{10}$ alkyl groups substituted with one, or more, azido, amino, alkylamino, dialkylamino, aralkylamino, halogen, hydroxy, carboxy, cyano, alkoxycarbonyl, alkanoyloxy, alkoxy, phenyloxy, aminocarbonyl, (substituted phenyl)oxy, (heteroaryl)oxy, mercapto, alkylthio, phenylthio, (substituted phenyl)thio, alkylsulfinyl or alkylsulfonyl,

substituted amino refers to a group having the formula —$NY_1Y_2$ wherein $Y_1$ is hydrogen, alkyl, phenyl, substituted phenyl, phenylalkyl or (substituted phenyl)alkyl, and $Y_2$ is alkyl, phenyl, substituted phenyl, phenylalkyl, (substituted phenyl)alkyl, hydroxy, cyano, alkoxy, phenylalkoxy, or amino (—$NH_2$).

The terms "alkyl", "alkoxy" and "alkanoyl", "alkenyl", "alkynyl", "alken-1-yl" and "alkyn-1-yl" refer to both straight and branched chain groups.

The terms "cycloalkyl" refer to cycloalkyl groups having 3, 4, 5, 6, or 7 carbon atoms.

The term "halogen" refers to fluorine, chlorine, bromine and iodine.

The term "protected carboxyl" refers to a carboxyl group which has been esteified with a conventional acid protecting group. These groups are well known in the art; see, for example, US—A—4,144,333, (issued

March 13, 1979). The preferred protected carboxyl groups are benzyl, benzhydryl, *t*-butyl, and *p*-nitrobenzyl esters.

Exemplary aromatic heterocyclic groups are substituted and unsubstituted pyridinyl, furanyl, pyrrolyl, thienyl, 1,2,3-triazolyl, 1,2,4-triazolyl, imidazolyl, thiazolyl, pyrimidinyl, oxazolyl, 1,3,4-thiazolyl and tetrazolyl. Exemplary of fully or partially saturated heterocyclic groups are substituted and unsubstituted piperidinyl, piperazinyl, 1-imidazolidinyl-2-one, and 1-piperazinyl-2,3,-dione.

The term "acyl derived from a carboxylic acid" includes all organic radicals derived from a carboxylic acid by removal of the hydroxyl group. Certain acyl groups are, of course, preferred. Exemplary acyl groups are those acyl groups which have been used in the past to acylate β-lactam antibiotics including 6-aminopenicillanic acid and derivatives and 7-aminocephalosporanic acid and derivatives; see, for example, *Cephalosporins and Penicillins,* edited by Flynn, Academic Press (1972), DE—A—2,716,677, (published October 10, 1978), BE—A—867,994, (published December 11, 1978), US—A—4,152,432, (issued May 1, 1979), US—A—3,971,778, (issued July 27, 1976), US—A—4,172,199, (issued October 23, 1979), and GB—A—1,348,894, (published March 27, 1974). The portions of these references describing various acyl groups are incorporated herein by reference. The following list of acyl groups is presented to further exemplify the term "acyl". Exemplary acyl groups are:

(a) Aliphatic groups having the formula

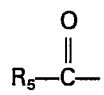

wherein $R_5$ is alkyl; cycloalkyl; alkoxy; alkenyl; cycloalkenyl; cyclohexadienyl; or alkyl or alkenyl substituted with one or more halogen, cyano, nitro, amino, mercapto, alkylthio, or cyanomethylthio groups.

(b) carbolic aromatic groups having the formula

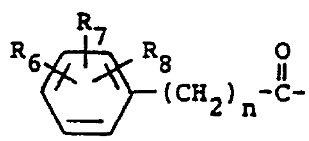

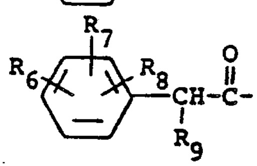

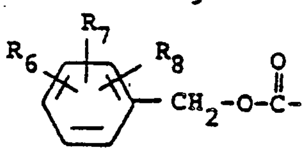

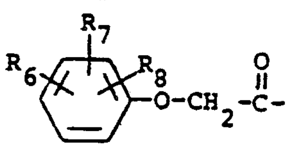

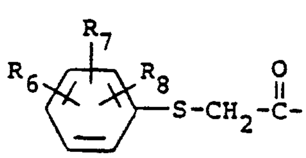 or

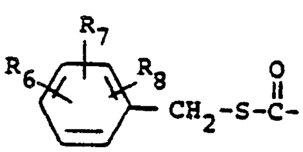

wherein n is 0, 1, 2 or 3; $R_6$, $R_7$, and $R_8$ each is independently hydrogen, halogen, hydroxyl, nitro, amino, cyano, trifluoromethyl, alkyl of 1 to 4 carbon atoms, alkoxy of 1 to 4 carbon atoms or aminomethyl; and $R_9$ is amino, hydroxyl, a carboxyl salt, protected carboxyl, formyloxy, a sulfo salt, a sulfoamino salt, azido, halogen, hydrazino, alkylhydrazino, phenylhydrazino, or [(alkylthio)thioxomethyl]thio.

Preferred carbocyclic aromatic acyl acyl groups include those having the formula

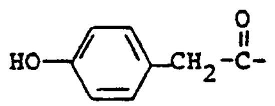

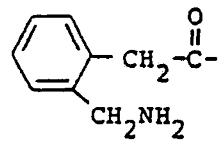

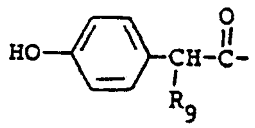

($R_9$ is preferably a carboxyl salt or sulfo salt) and

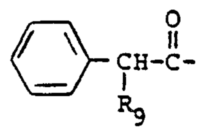

($R_9$ is preferably a carboxyl salt or sulfo salt).

(c) Heteroaromatic groups having the formula

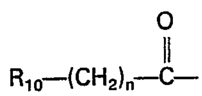

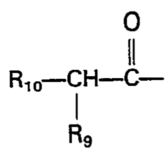

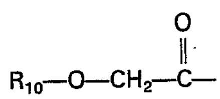

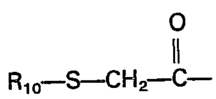 or

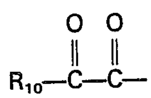

wherein n is 0, 1, 2 or 3; $R_9$ is as defined above; and $R_{10}$ is a substituted or unsubstituted 5-, 6- or 7-membered heterocyclic ring containing 1, 2, 3 or 4 (preferably 1 or 2) nitrogen, oxygen and sulfur atoms. Exemplary heterocyclic rings are thienyl, furyl, pyrrolyl, pyridinyl, pyrazolyl, pyrazinyl, thiazolyl, pyrimidinyl and tetrazolyl. Exemplary substituents are halogen, hydroxyl, nitro, amino, cyano, trifluoromethyl, alkyl of 1 to 4 carbon atoms, alkoxy of 1 to 4 carbon atoms, or

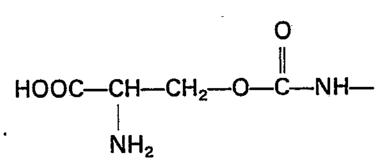

Preferred heteroaromatic acyl groups include those groups of the above formulas wherein $R_{10}$ is 2-amino-4-thiazolyl, 2-amino-5-halo-4-thiazolyl, 4-aminopyrimidin-2-yl, 5-amino-1,2,4-thiadiazol-3-yl, 2-thienyl, 2-furanyl, or 6-aminopyridin-2-yl.

(d) [[(4-Substituted-2,3-dioxo-1-piperazinyl)carbonyl]amino]arylacetyl groups having the formula

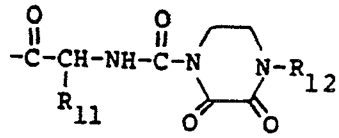

wherein $R_{11}$ is an aromatic group (including carbocyclic aromatics such as those of the formula

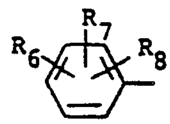

and heteroaromatics as included within the definition of $R_{10}$); and $R_{12}$ is alkyl, substituted alkyl (wherein the alkyl group is substituted with one or more halogen, cyano, nitro, amino or mercapto groups), arylmethyleneamino (*i.e.,* —N=CH—$R_{11}$ wherein $R_{11}$ is as defined above), arylcarbonylamino (*i.e.,*

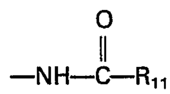

wherein $R_{11}$ is as defined above) or alkylcarbonylamino.

Preferred [[(4-substituted-2,3-dioxo-1-piperazinyl)carbonyl]amino]arylacetyl groups include those wherein $R_{12}$ is ethyl, phenylmethyleneamino or 2-furylmethyleneamino.

(e) (Substituted oxyimino)arylacetyl groups having the formula

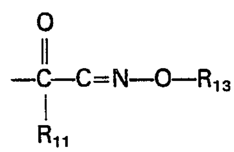

wherein $R_{11}$ is as defined above and $R_{13}$ is hydrogen, alkyl, cycloalkyl, alkylaminocarbonyl, arylaminocarbonyl (*i.e.,*

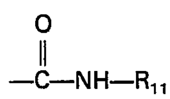

wherein $R_{11}$ is as defined above) or substituted alkyl (wherein the alkyl group is substituted with 1 or more halogen, cyano, nitro, amino, mercapto, alkylthio, aromatic group (as defined by $R_{11}$), carboxyl (including salts thereof), amido, alkoxycarbonyl, phenylmethoxycarbonyl, diphenylmethoxycarbonyl, hydroxyalkoxyphosphinyl, dihydroxyphosphinyl, hydroxy (phenylmethoxy)phosphinyl, or dialkoxyphosphinyl substituents).

Preferred (substituted oxyimino)arylacetyl groups include those wherein $R_{11}$ is 2-amino-4-thiazolyl. Also preferred are those groups wherein $R_{13}$ is methyl, ethyl, carboxymethyl, 1-carboxy-1-methylethyl or 2,2,2,-trifluoroethyl.

(f) (Acylamino)arylacetyl groups having the formula

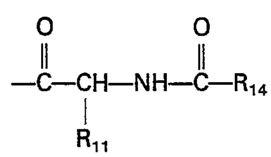

wherein $R_{11}$ is as defined above and $R_{14}$ is

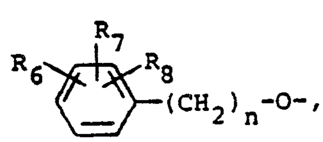

amino, alkylamino, (cyanoalkyl)amino, amido, alkylamido, (cyanoalkyl)amido

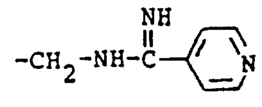 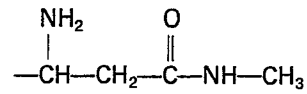

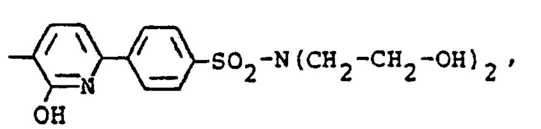 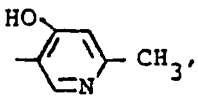

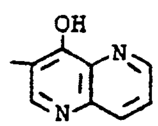, or 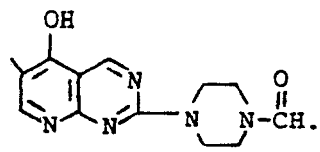

Preferred (acylamino)arylacetal groups of the above formula include those groups wherein $R_{14}$ is amino or amido. Also preferred are those groups wherein $R_{11}$ is phenyl or 2-thienyl.

(g) [[[3-Substituted-2-oxo-1-imidazolidinyl]carbonyl]amino]arylacetyl groups having the formula

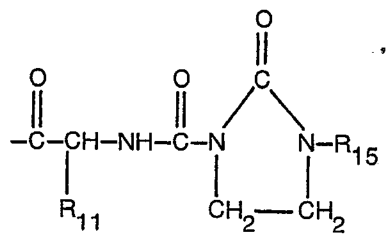

wherein $R_{11}$ is as defined above and $R_{15}$ is hydrogen, alkylsulfonyl, arylmethyleneamino (*i.e.,* $-N=CH-R_{11}$ is as defined above),

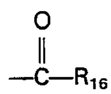

(wherein $R_{16}$ is hydrogen, alkyl or halogen substituted alkyl), aromatic group (as defined by $R_{11}$ above), alkyl or substituted alkyl (wherein the alkyl group is substituted with one or more halogen, cyano, nitro, amino or mercapto groups).

Preferred [[3-substituted-2-oxo-1-imidazolidinyl]carbonyl]amino]arylacetyl groups of the above formula include those wherein $R_{11}$ is phenyl or 2-thienyl. Also preferred are those groups wherein $R_{15}$ is hydrogen, methylsulfonyl, phenylmethyleneamino or 2-furylmethyleneamino.

The compounds of this invention form basic salts with various inorganic and organic bases which are also within the scope of this invention. Such salts include ammonium salts, alkali metal salts, alkaline earth metal salts, salts with organic bases, *e.g.,* dicyclohexylamine, benzathine, N-methyl-D-glucamine, hydrabamine and the like. The pharmaceutically acceptable salts are preferred, although other salts are also useful, *e.g.,* in isolating or purifying the product.

Some of the compounds of this invention may be crystallized or recrystallized from solvents containing water. In these cases water of hydration may be formed. This invention contemplates stoichiometric hydrates as well as compounds containing variable amounts of water that may be produced by processes such as lyophilization.

β-Lactams having a

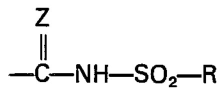

substituent in the 1-position and an amino or acylamino substituent in the 3-position contain at least one chiral center — the carbon atom (in the 3-position of the β-lactam nucleus) to which the amino or acylamino substituent is attached. This invention is directed to those β-lactams which have been described above, wherein the stereochemistry at the chiral center in the 3-position of the β-lactam nucleus is the same as the configuration at the carbon atom in the 6-position of naturally occurring penicillins (*e.g.,* penicillin G) and as the configuration at the carbon atom in the 7-position of naturally occurring cephamycins (*e.g.,* cephamycin C).

With respect to the β-lactams of formulas (I), (II) and (III), the structural formulas have been drawn to show the stereochemistry at the chiral center in the 3-position.

Also included within the scope of this invention are racemic mixtures which contain the above-described β-lactams.

β-Lactams of formula (I) and salts thereof, have activity against a range of gram-negative and gram-positive organisms. The compounds are highly active against a range of aerobic gram-negative microorganisms including, for example, strains of *Escherichia coli, Klebsiella* sp., *Proteus* sp., *Enterobacter* sp., *Serratia marcescens* and *Pseudomonas aeruginosa.* The compounds show good stability to β-lactamases, for example, those enzymes produced by aerobic gram-negative organisms and classified into Groups I—V by Richmond & Sykes, "Advances in Microbial Physiology" (Rose et al., eds.), Vol. *9*, 31, Academic Press, London & New York.

The compounds of this invention can be used as agents to combat bacterial infections (including urinary tract infections and respiratory infections) in mammalian species, such as domesticated animals (*e.g.,* dogs, cats, cows, horses, and the like) and humans.

For combating bacterial infections in mammals a compound of this invention can be administered to a mammal in need thereof in an amount of 1.4 mg/kg/day to 350 mg/kg/day, preferably 14 mg/kg/day to 100 mg/kg/day. All modes of administration which have been used in the past to deliver penicillins and cephalosporins to the site of the infection are also contemplated for use with the novel family of β-lactams of this invention. Such methods of administration include oral, intravenous, intramuscular and as a suppository.

A

$$-\overset{\overset{\displaystyle Z}{\|}}{C}-NH-SO_2-R$$

activating group can be introduced onto the nitrogen atom of a β-lactam by conventional methods as by reacting the β-lactam with the appropriate isocyanate having the formula (VIIa)

$$O{=}C{-}N{-}SO_2{-}R \qquad \text{VIIa}$$

or with the appropriate isothiocyanate having the formula (VIIb)

$$S{=}C{=}N{-}SO_2{-}R \qquad \text{VIIb}$$

The reaction is preferably run in an organic solvent, *e.g.,* in an inert solvent such as tetrahydrofuran or dimethoxyethane, in the presence of a base such as triethylamine or alkyl lithium.

An alternative, and preferred route for introducing a

$$-\overset{\overset{\displaystyle Z}{\|}}{C}-NH-SO_2-R$$

substituent onto the nitrogen atom of a β-lactam when R is an amino or substituted amino group, comprises first reacting the β-lactam with an isocyanate having the formula (VIIIa)

$$O{=}C{=}N{-}SO_2{-}Y \qquad \text{VIIIa}$$

or with the appropriate isothiocyanate having the formula (VIIIb)

$$S{=}C{=}N{-}SO_2{-}Y \qquad \text{VIIIb}$$

wherein Y is a leaving group, *e.g.,* a halogen such as chlorine. The reaction is preferably run in an inert organic solvent, *e.g.,* a halocarbon such as dichloromethane or in acetonitrile. The resulting intermediate has a

$$-\overset{\overset{\displaystyle Z}{\|}}{C}-NH-SO_2-Y$$

substituent on the nitrogen atom of the β-lactam. Displacement of the leaving group ("Y") with e.g. the desired $-NR_aR_b$ group can be accomplished with the appropriate nucleophile having the formula $HNR_aR_b$. Alternatively, the displacement of the leaving group ("Y") can be accomplished by reaction with a protected form of the compound $HNR_aR_b$, such as the appropriate silyl protected compound, followed by hydrolysis of the silyl group or groups.

The β-lactams of formula I can be prepared from a 3-protected amino-2-azetidinone having the formula (IX)

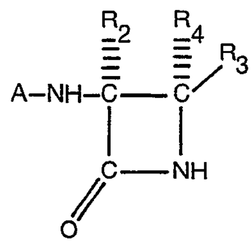

IX

In formula (IX), and throughout the specification, the symbol "A" refers to an amino protecting group. These groups are well known in the field of β-lactam chemistry, and the particular group chosen is not critical. Benzyloxycarbonyl, trityl, and *t*-butoxycarbonyl are exemplary protecting groups.

The addition of a

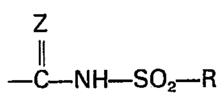

activating group to a compound of formula (IX) (using the procedure described above) yields a compound having the formula (X)

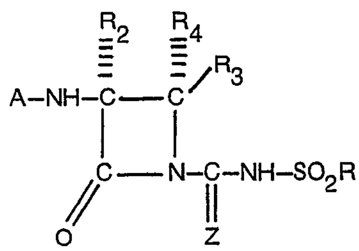

X

Deprotection of a compound of formula (X) using conventional techniques yields the corresponding key intermediate having the formula (III)

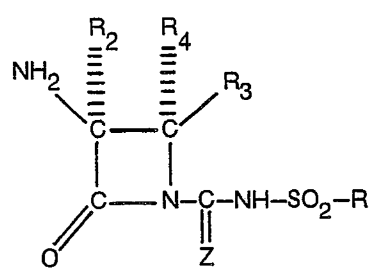

III

or a salt thereof.

The particular deprotection reaction used will, of course, depend on the protecting group ("A") present. If, for example, A is a *t*-butoxycarbonyl protecting group, deprotection can be accomplished by treatment of a compound of formula VI with acid (*e.g.,* formic acid or trifluoroacetic acid). If, for example, A is a benzyloxycarbonyl protecting group, deprotection can be accomplished by catalytic hydrogenation of a compound of formula (X).

Well known acylation techniques can be used to convert an intermediate of formula (III) to a corresponding product of formula (I). Exemplary techniques include reaction of a compound of formula (III) with a carboxylic acid ($R_1$—OH), or corresponding carboxylic acid halide or carboxylic acid anhydride. The reaction with a carboxylic acid proceeds most readily in the presence of a carbodiimide such as dicyclohexylcarbodiimide and a substance capable of forming an active ester *in situ* such as N-hydroxybenzotriazole. In those instances where the acyl group ($R_1$) contains reactive functionality (such as amino or carboxyl groups) it may be necessary to first protect those functional groups, then carry out the acylation reaction, and finally deprotect the resulting product.

An alternative procedure for preparing the compounds of formula (I) comprises first acylating (acylation techniques have been described above) a 3-amino-2-azetidinone having the formula (XI)

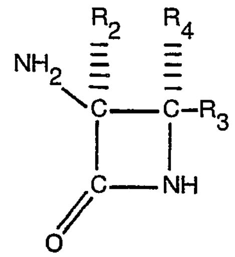

XI

to yield an intermediate having the formula (XII)

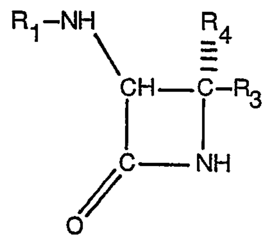

XII

A

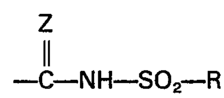

activating group can be introduced in the 1-position of a compound of formula (XII) (using the procedures described above) to obtain the corresponding product of formula (I). In those instances wherein the acyl side-chain "$R_1$" contains reactive functionality (such as amino groups), it may be necessary to first protect those functional groups, then carry out the addition of the activating group in the 1-position, and finally deprotect the resulting product.

Still another synthesis for the preparation of compounds of formula (I) wherein $R_2$ is hydrogen comprises the use of a 3-azido-2-azetidinone having the formula (XIII)

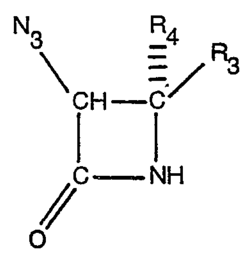

XIII

A

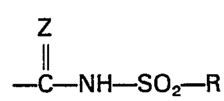

activating group can be introduced in the 1-position of a compound of formula (XIII) (using the procedures described above) to obtain the corresponding compound having the formula (XIV)

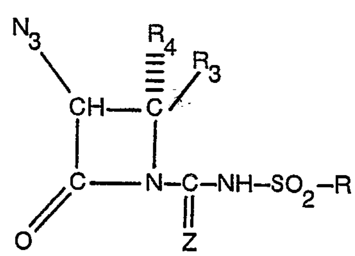

XIV

The compounds of formula XIV are novel intermediates, and as such, they constitute an integral part of this invention.

Reduction of an intermediate of formula (XIV) yields the corresponding intermediate having the formula (XV)

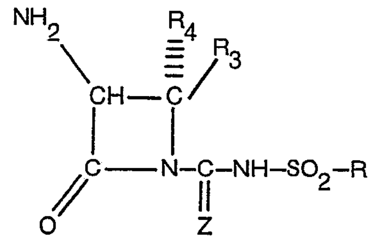

XV

The reduction can be accomplished by catalytic (*e.g.,* palladium on charcoal or platinum oxide) hydrogenation or with reducing agents such as zinc or triphenylphosphine. As described above, from these key intermediates (compounds of formula III), using conventional acylation techniques, it is possible to prepare all of the products of formula I wherein $R_2$ is hydrogen.

Example 1

(S)-[1-[[[(4-Methylphenyl)sulfonyl]amino]carbonyl]-2-oxo-3-azetidinyl]carbamic acid, phenylmethyl ester

A) 1-[(1R)-Carboxy-2-methyl(propyl)]-2-oxo-(3S)-[[(phenylmethoxy)carbonyl]amino]azetidine

A slurry of 6-aminopenicillanic acid (12.98 g) in 140 ml of water containing 5.18 g of sodium bicarbonate (stirred for about 10 minutes without complete solution) is added in one portion to a well-stirred (mechanical stirrer) suspension of Raney nickel (washed with water to pH 8.0, 260 ml of slurry = 130 g) in a 70°C oil bath. After 15 minutes the slurry is cooled, filtered, and the filtrate treated with 5.18 g of sodium bicarbonate and a solution of 11.94 g of benzyl chloroformate in 12 ml of acetone. After 30 minutes, the solution is acidified to pH 2.5 and extracted with methylene chloride. The organic layer is dried, evaporated, and triturated with ether-hexane to give a total of 6.83 g of the title compound.

B) 1-[(Acetyloxy)-2-methyl(propyl)]-2-oxo-(3S)-[[(phenylmethoxy)carbonyl]amino]azetidine

A solution of 6.83 g of 1-[(1R)-carboxy-2-methyl(propyl)]-2-oxo-(3S)-[[(phenylmethoxy)carbonyl]-amino]azetidine in 213 ml of of acetonitrile is treated with 1.95 g of cupric acetate monohydrate and 9.5 g of lead tetraacetate.

The slurry is immersed in a 65°C oil bath and stirred with a stream of nitrogen bubbling through the slurry until the starting material is consumed. The slurry is filtered and the solids washed with ethyl acetate. The combined filtrate and washings are evaporated *in vacuo* and the residue is taken up in 100 ml each of ethyl acetate and water and adjusted to pH 7. The ethyl acetate layer is separated, dried, and evaporated to give 6.235 g of the title compound.

C) (S)-(2-Oxo-3-azetidinyl)carbamic acid, phenylmethy ester

A solution of 3.12 g of 1-[(acetyloxy)-2-methyl(propyl)]-2-oxo-(3S)-[[(phenylmethoxy)carbonyl]amino]-azetidine in 70 ml of methanol and 7 ml of water is cooled to −15°C and 1.33 g of potassium carbonate and 349 mg of sodium borohydride are added. The reaction mixture is stirred at −15°C — 0°C. After the reaction is complete (about 2 hours), the mixture is neutralized to pH 7 with 2N HCl and concentrated *in vacuo.* The concentrate is adjusted to pH 5.8, saturated with salt and extracted with ethyl acetate (3 times). The organic layer is dried and evaporated *in vacuo*. The residue is combined with material from a similar experiment and triturated with ether to give 3.30 g of the title compound.

D) (S)-[1-[[[(4-Methylphenyl)sulfonyl]amino]carbonyl]-2-oxo-3-azetidinyl]carbamic acid, phenylmethyl ester, triethylammonium salt

(S)-(2-Oxo-3-azetidinyl)carbamic acid, phenylmethyl ester (220 mgs) is dissolved in dry tetrahydrofuran (10 ml) and stirred well under nitrogen. Triethylamine (139 µl) is added, followed by *p*-toluenesulfonyl isocyanate (153 µl, 1 mmol). After 2 to 3 hours of stirring a crystalline material starts to precipitate out. After 2 hours of further stirring, the solid is removed by filtration, washed with a small volume of acetonitrile and dried *in vacuo.* Yield of the triethylamine salt is 332 mg, melting point 128—130°C.

E) (S)-[1-[[[(4-Methylphenyl)sulfonyl]amino]carbonyl]-2-oxo-azetidinyl]carbamic acid, phenylmethyl ester

The triethylamine salt above (345 mg) is dissolved in water (15 ml) and layered with ethyl acetate (15 ml). The mixture is acidified to pH 2.0 and well shaken. The extraction with ethyl acetate is repeated twice. The combined ethyl acetate layers are dried over anhydrous sodium sulfate and evaporated to dryness *in vacuo* to yield 263 mg of a gum. This is crystallized from dichloromethane/hexane yielding 131 mg of product, melting point 178—180°C.

Analysis for $C_{19}H_{19}O_6N_3S$:
Calc'd: C, 54.67; H, 4.59; N, 10.07; S, 7.68
Found: C, 54.49; H, 4.71; N, 10.05; S, 7.75

Example 2

(S)-[1-[[[(4-Methylphenyl)sulfonyl]amino]carbonyl]-2-oxo-3-aminoazetidine, triethylammonium salt

(S)-[1-[[[(4-Methylphenyl)sulfonyl]amino]carbonyl]-2-oxo-3-azetidinyl]carbamic acid, phenylmethyl ester, triethylammonium salt (150 mg; see example 1) is dissolved in dry methanol (10 ml). 10% Palladium on carbon (75 mg) is added and the mixture is hydrogenated for 30 minutes. The catalyst is removed by filtration and the filtrate is evaporated *in vacuo* to dryness, yielding 81 mg of the title compound.

Example 3

(S)-N-[(4-Methylphenyl)sulfonyl]-2-oxo-3-[(phenylacetyl)amino]-1-azetidinecarboxamide

(S)-[1-[[[(4-Methylphenyl)sulfonyl]amino]carbonyl]-2-oxo-3-aminoazetidine, triethylammonium salt (626 mg, see example 2) is dissolved in dry acetonitrile (25 ml) and stirred under nitrogen in an ice bath. Triethylamine (.57 ml) is added, followed by phenylacetyl chloride (.324 ml). After 3 hours an equal volume of water is added, the pH is adjusted to 7.5, and the acetonitrile is removed *in vacuo*. The aqueous residue is extracted twice with ethyl acetate to remove neutral material. The aqueous layer is then acidified to pH 2.0 and extracted three times with ethyl acetate. The acidic extract is dried over anhydrous sodium sulfate and evaporated to dryness *in vacuo,* yielding 544 mg. This crude product, and an additional 481 mg of crude product from two other runs are combined and purified by chromatography on a column of SilicAR CC-4 (100 g), using dichloromethane and dichloromethane:methanol, 99:1 as eluants. The amorphous product is crystallized from acetonitrile to give 154 mg of crystalline material, melting point 170—172°C.

| | | | | |
|---|---|---|---|---|
| Analysis for $C_{19}H_{19}N_3O_5S$: | C, 56.85, | H, 4.77; | N, 10.47; | S, 7.99 |
| Found: | C, 56.70; | H, 4.91; | N, 10.41; | S, 8.05 |

Example 4

[3S(Z)]-3-[[(2-Amino-4-thiazolyl)(methoxyimino)acetyl]amino]-N-[(4-methylphenyl)sulfonyl]-2-oxo-1-azetidinecarboxamide

A) (S)-3-Amino-2-azetidinone

(S)-Oxo-3-azetidinyl)carbamic acid, phenylmethyl ester (3 g; see example 1C) is hydrogenated in 100 ml of methanol in the presence of 1 g of palladium on charcoal catalyst. When the theoretical amount of hydrogen is absorbed, the catalyst is filtered off and the filtrate evaporated to dryness. On standing, 1.1 g of the title compound crystallizes.

B)[3S(Z)]-3-[[[2-(Triphenylmethylamino)-4-thiazolyl][methoxyimino]acetyl]amino]-2-oxoazetidine

(Z)-2-(Triphenylmethylamino)-α-(methoxyimino)-4-thiazoleacetic acid (2.50 g) is dissolved in dry dimethylformamide (25 ml). 1-Hydroxybenzotriazole (863 mg, 5.64 mmol), N,N'-dicyclohexylcarbodiimide (1.164 g) and 3-amino-2-oxoazetidine (485 mg) are added sequentially, and the mixture is stirred at room temperature under dry nitrogen for 5 hours. The reaction mixture is worked up by diluting it with water (250 ml) adjusting to pH 7.5, and extracting (three times) with an equal volume of ethyl acetate. The combined extract is washed with water followed by saturated aqueous sodium chloride, dried over anhydrous sodium sulfate, and evaporated to dryness *in vacuo.* The crude product is purified by chromatography over SilicAR CC—7 silica gel, to give 2.50 g of solid. This is crystallized from chloroform-hexane to yield 2.31 g of the title compound, melting point 233—236°C.

C) [3S(Z)]-3-[[[2-Triphenylmethylamino)-4-thiazolyl][methoxyimino]acetyl]amino]-N-[(4-methylphenyl)sulfonyl]-2-oxo-1-azetidinecarboxamide

[3S(Z)]-2-[[[2-(Triphenylmethylamino)-4-thiazolyl][methoxyimino]acetyl]amino]-2-oxo-1-azetidine (270 mg) is dissolved in dry tetrahydrofuran (5 ml). Triethylamine (66 µl) is added followed by *p*-toluenesulfonyl isocyanate (80 µl). The mixture is stirred for 16 hours at room temperature. Following removal of the solvent *in vacuo* the residue is taken up in ethyl acetate/water and adjusted to pH 7.5. The extraction with ethyl acetate is repeated twice. The combined organic extracts are washed with water, dried over anhydrous sodium sulfate, and evaporated to dryness *in vacuo* to give 263 mg of crude product, which is purified by chromatography on SilicAR CC—4 silic gel (27 g), using dichloromethane:ethyl acetate (3:1), to give 123 mg of the title compound.

D)[3S(Z)]-3-[[(2-Amino-4-thiazolyl)(methoxyimino)acetyl]amino]-N-[(4-methylphenyl)sulfonyl]-2-oxo-1-azetidinecarboxamide

[3S(Z)] - 3 - [[[2-(Triphenylmethylamino) - 4 - thiazolyl][methoxyimino]acetyl]amino] - N - [(4 - methylphenyl)sulfonyl] - 2 - oxo - 1 - azetidinecarboxamide (122 mg) is dissolved in 70% aqueous formic acid (2 ml) and stirred under nitrogen at room temperature for 3 hours. After removal of the excess acid *in vacuo* the residue is taken up in ethyl acetate/water at pH 7.5. The organic layer is removed. The aqueous layer is acidified to pH 2.5 and extracted (five times) with ethyl acetate. The extract is dried over anhydrous sodium sulfate and evaporated to dryness *in vacuo.* The residue is dissolved in pure dioxane and lyophilized to yield 50 mg of the title compound as a fluffy powder, which is dried *in vacuo* at 50°C for 2 hours, melting point 160°C *dec.*

Analysis for $C_{17}H_{18}O_6N_6S_2$:
Calc'd: C, 43.78; H, 3.89; N, 18.02; S, 13.75
Found: C, 43.38; H, 4.05; N, 15.86; S, 12.75

Example 5

(S)-N-(Methylsulfonyl)-2-oxo-3-[[(phenylmethoxy)carbonyl]amino]-1-azetidinecarboxamide

A stirred suspension of (S)-(2-oxo-3-azetidinyl)carbamic acid, phenylmethyl ester (2.20 g; see example 1C) in 60 ml of dry tetrahydrofuran under nitrogen is warmed using a water bath at 40°C until solution occurs. The solution is cooled to −75°C, and 7.4 ml of 1.35 M sec-butyl lithium in cyclohexane (10 mmol) is added. After stirring for 2 minutes, (methylsulfonyl) isocyanate (about 1.20 g) is added. This reaction is stirred at −75°C for 10 minutes and poured into 100 ml of 0.5 M pH 5.5 monobasic potassium phosphate buffer. Ethyl acetate is added, and the pH is adjusted to 7. After several extractions with ethyl acetate, the aqueous layer is covered with ethyl acetate and adjusted to pH 2. Repeated extraction with ethyl acetate gives an acidic extract, which is dried (sodium sulfate) and evaporated to a residue (2.61 g). Chromatography of this residue on 250 g of SilicAR CC—4 using dichloromethane and then 1% methanol in dichloromethane provides 1.82 g of product as a residue.

Example 6

(S)-N-(Methylsulfonyl)-2-oxo-3-amino-1-azetidinecarboxamide, hydrochloride salt

To a solution of 300 mg of (S)-N-(methylsulfonyl)-2-oxo-3-[[(phenylmethoxy)carbonyl]amino-1-azetidinecarboxamide (see example 5) in 8 ml of dry methanol is added 150 mg of 10% palladium on charcoal. The mixture is hydrogenated at room temperature and 1 atmosphere for 30 minutes, and then 2 ml of water is added followed by dilute hydrochloric acid to adjust the pH to 1.8. The catalyst is filtered using water/methanol. Evaporation of the filtrate gives 65 mg of crude product. The catalyst is suspended in water, adjusted to pH 1.8 and stirred at room temperature for 15 minutes. Removal of the catalyst and evaporation of the filtrate provides additional crude product (124 mg).

Example 7

(S)-N-(Methylsulfonyl)-2-oxo-3-[(phenylacetyl)amino]-1-azetidinecarboxamide

(S)-N-(Methylsulfonyl)-2-oxo-3-amino-1-azetidinecarboxamide, hydrochloride salt (189 mg; see example 6) is suspended in 8 ml of dry acetonitrile and 0.54 ml of triethylamine, and stirred at room temperature under nitrogen until solution occurs (about 15 minutes). The solution is cooled to 0 to 5°C, and then phenyl acetylchloride (153 µl) is added. The reaction is stirred at room temperature for 3 hours and then poured into 10 ml of 0.5 M pH 5.5 monobasic potassium phosphate buffer. The pH is adjusted to 7 (dilute potassium hydroxide) and the solution is extracted twice with ethyl acetate. The aqueous layer is covered with fresh ethyl acetate, and the pH is adjusted to 2 (3N hydrochloric acid). Repeated extraction with ethyl acetate gives an acidic ethyl acetate extract, which is dried (sodium sulfate) and evaporated to a residue (196 mg). Chromatography of this material on 20 g of SilicAR CC—4 using ethyl acetate/dichloromethane (1:1) provides 96 mg of crystalline product after removal of solvent. Recrystallization from ethyl acetate/dichloromethane provides an analytical sample having a melting point 168—170°C, *dec.*

Anal. Calc'd for $C_{13}H_{15}N_3O_5S$: C, 48.00; H, 4.65; N, 12.92; S, 9.84.
Found: C, 47.82; H, 4.74; N, 12.86; S, 9.13

Example 8

(S)-3-Amino-N-(methylsulfonyl)-2-oxo-1-azetidinecarboxamide, inner salt

(S)-3-N-(Methylsulfonyl)-2-oxo-1-3-[[(phenylmethoxy)carbonyl]amino]-1-azetidinecarboxamide (2.20 g; see examaple 5) is stirred with 90 ml of methanol under nitrogen at 40 to 45°C until solution occurs. The solution is quickly cooled to room temperature and hydrogenated at one atmosphere in the presence of 1.1 g of 10% palladium on charcoal for twenty minutes. Water (20 ml) is added, and the pH is adjusted to 2.0 (1N hydrochloric acid). The product, which is absorbed on the catalyst, is collected by filtration, using Whatman #50 paper, and suspended in d.23950 paper, and suspended in 25 ml of water. The pH is adjusted to 2.0 (1N hydrochloric acid), and the mixture is stirred for twenty-five minutes and filtered through Whatman #50 paper. Evaporation of the filtrate provides 611 mg of crystalline product, melting point 180°C, *dec.*

Example 9

[3S(Z)]-3-[[(2-Amino-4-thiazolyl)(methoxyimino)acetyl]amino]-N-(methylsulfonyl)-2-oxo-1-azetidinecarboxamide, potassium salt

Triethylamine (0.47 ml) is added to a stirred suspension of (Z)-(2-amino-4-thiazolyl)methoxyimino)acetic acid (671 mg) and 2 µl of N-methylmorpholine in 5 ml of dry dimethylformamide at room temperature under nitrogen. The mixture is stirred for five minutes and then cooled to −25°C. Diphenyl chlorophosphate (0.69 ml) is added, and the mixture is stirred at −25° to −15°C for forty minutes. Using a syringe, this mixture is transferred to a stirred mixture of (S)-3-amino-N-(methylsulfonyl)-2-oxo-1-azetidinecarboxamide, inner salt (608 mg; see example 8), 5 ml of dry

dimethylformamide and 1.65 ml (11.8 mmol) of triethylamine at −25°C under nitrogen. After stirring at −25° to −15°C for three and a half hours, the reaction mixture is poured into 35 ml of 0.5 M, pH 5.5, monobasic potassium phosphate. Water and ethyl acetate are added, and the pH is adjusted to 7.2 using dilute potassium hydroxide. After extracting with ethyl acetate (three times), the aqueous layer is covered with fresh ethyl acetate and adjusted to pH 2.5 (1N hydrochloric acid). Repeated extraction with ethyl acetate gives an acidic ethyl acetate extract, which is dried (sodium sulfate), and evaporated to a residue (625 mg).

This residue is applied to a column of SilicAR CC—4 (50 g) packed in ethyl acetate/dichloromethane (1:1). Elution with this solvent and then ethyl acetate removes non-polar impurities. Elution with 3—4% methanol in ethyl acetate provides 334 mg of partially purified product ($H^+$ form), which was taken up in 3 ml of 0.5 M ph 5.5 monobasic potassium phosphate buffer. The pH is adjusted to 6.5 (dilute potassium hydroxide), and the solution is passed, using water, through a column of HP—20 resin (100 ml), which has been prewashed with the monobasic potassium buffer and then water. A combination of phosphomolybdic acid test ($PO_4^{-3}$) and Rydon's test locates the desired product. Evaporation of appropriate fractions provides 145 mg of desired potassium salt as a residue.

The aqueous pH 2.5 layer mentioned above, is evaporated to remove water and dimethylformamide. Water and ethyl acetate are added, and after adjusting the pH to 2.5, another acidic ethyl acetate extract is obtained and evaporated to a residue (658 mg). This material is dissolved in 4 ml and 0.5 M monobasic potassium phosphate buffer and adjusted to pH 6.5. Subsequent passage through 100 ml of HP—20 resin using water provides 54 mg of additional potassium salt as a residue. This material is combined with the 145 mg portion above and lyophilized from water to give 199 mg of the title potassium salt as a solid, melting point 205°C, *dec.*

| | | | | |
|---|---|---|---|---|
| Anal. Cal'cd. for $C_{11}H_{13}N_6O_6S_2K \cdot 2\ H_2O$: | C, 28.45; | H, 3.69; | N, 18.10; | S, 13.78 |
| Found: | C, 28.75; | H, 2.87; | N, 18.09; | S, 13.64 |

## Example 10

### [3S-[3α,4β]]-3-[[(1,1-Dimethylethoxy)carbonyl]amino]-4-methyl-N-(methylsulfonyl)-2-oxo-1-azetidinecarboxamide

A) N-Benzyloxy-*t*-butoxycarbonyl-threonine amide

A solution of 8.76 g of *t*-butoxycarbonyl threonine and the free amine from 6.4 g of O-benzylhydroxylamine HCl (ethyl acetate-sodium bicarbonate liberation) in 100 ml of tetrahydrofuran is treated with 6.12 g of N-hydroxybenzotriazole and 8.24 g of dicyclohexylcarbodiimide in 20 ml of tetrahydrofuran. The mixture is stirred under nitrogen for 26 hours, filtered, and evaporated *in vacuo*. The residue is chromatographed on a 300 g silica gel column (elution with chloroform and chloroform/ethyl acetate (3:1) yielding 7.2 g of compound. Crystallization from ether-hexane gives 4.18 g of the title compound.

B) (3S-*trans*)-N-Benzyloxy-3-*t*-butoxycarbonylamino-4-methylazetidinone

A solution of 12.67 g of N-benzyloxy-*t*-butoxycarbonyl-threonine amide, 11.5 g of triphenylphosphine, and 6.23 ml of diethylazodicarboxylate in 380 ml of tetrahydrofuran is stirred under nitrogen for about 16 hours. The solution is evaporated and chromatographed on a 900 gram silica gel column. Elution with chloroform-ethyl acetate (3:1) gives 13.69 g of compound that crystallises from ether-hexane to yield 9.18 g of the title compound.

C) (3S-*trans*)-3-*t*-Butoxycarbonylamino-1-hydroxy-4-methylazetidinone

A solution of 9.18 g of (3S-*trans*)-N-benzyloxy-3-*t*-butoxycarbonylamino-4-methylazetidinone in 300 ml of 95% ethanol is stirred in an atmosphere of hydrogen with 1.85 g of 10% palladium on charcoal. After 141 minutes the slurry is filtered and evaporated *in vacuo*. The residue is recrystallized from ether-hexane to yield 5.12 g of the title compound.

D) (3S-*trans)*-3-*t*-Butoxycarbonylamino-4-methylazetidnone

A solution of 4.98 g of (3S-*trans*)-3-*t*-butoxycarbonylamino-1-hydroxy-4-methylazetidinone in 200 ml of methanol is treated with 132 ml of 4.5 M ammonium acetate and then 66 ml of 1.5 M titanium trichloride and stirred for 4.5 hours. The aqueous solution is diluted with an equal volume of 8% sodium chloride and extracted with ethyl acetate to give 3.48 g of crude product. Recrystallization from ether-hexane yields 3.3 g of the title compound.

E) [3S-[3α,4β]]-3-[[(1,1-Dimethylethoxy)carbonyl]amino]-4-methyl-N-(methylsulfonyl)-2-oxo-1-azetidinecarboxamide

Sec-Butyl lithium (14.8 ml of 1.35 M solution in cyclohexane) is added to a stirred solution of (3S-*trans*)-3-*t*-butoxycarbonylamino-4-methylazetidinone (20 mmole) in 150 ml of dry tetrahydrofuran at −75°C under nitrogen. After stirring for 2 minutes methylsulfonylisocyanate (2.4 ml) is added, and the solution is stirred for 25 minutes at −75°C and poured into 200 ml of 0.5 M pH 5.5 monobasic potassium phosphate buffer. Water and ethyl acetate are added, and the pH is adjusted to 2.5 using 3 N hydrochloric acid. The ethyl acetate layer and subsequent ethyl acetate extract are combined, dried (sodium sulfate), and evaporated to

a residue (7.15 g), which is chromatographed on 450 g of SilicAR CC—4, using dichloromethane and then 1% methanol in dichloromethane, to give 3.67 g of desired product as a residue.

Example 11

[3S-[3α,4β]]-3-Amino-4-methyl-N-(methylsulfonyl)-2-oxo-1-azetidinecarboxamide, trifluoracetic acid salt

[3S - [3α,4β]] - 3 - [[(1,1 - Dimethylethoxy)carbonyl]amino] - 4 - methyl - N - (methylsulfonyl) - 2 - oxo - 1 - azetidinecarboxamide (482 mg; see example 10) is stirred with 4.5 ml of trifluoroacetic acid at 0 to 5°C under nitrogen for 30 minutes. The solution is evaporated *in vacuo* to a residue, which is evaporated from acetonitrile (four times) to give the desired salt as a residue.

Example 12

[3S-[3α(Z),4β]]-3-[[(2-Amino-4-thiazolyl)(methoxyimino)acetyl]amino]-4-methyl-N-(methylsulfonyl)-2-oxo-1-azetidinecarboxamide, potassium salt

Triethylamine (243 μl) is added to a stirred suspension of (Z)-(2-amino-4-thiazolyl)(methoxyimino)acetic acid (303 mg) in 4.5 ml of dry dimethylformamide at room temperature under nitrogen. After stirring for 2 minutes, the mixture is cooled to −25°C, and diphenyl chlorophosphate (312 μl) is added. The mixture is stirred for 50 minutes at −25°C and added, via syringe, to a stirred solution of [3S-[3α,4β]]-3-amino-4-methyl-N-(methylsulfonyl)-2-oxo-1-azetidinecarboxamide, trifluoroacetic acid salt and 1.05 ml of triethylamine in dimethylformamide at −25°C under nitrogen. The reaction is stirred for 2.5 hours at this temperature and poured into 18 ml of 0.5 M pH 5.5 monobasic potassium phosphate buffer. Ethyl acetate and water are added and the pH is adjusted to 7. The aqueous layer is separated and adjusted to 2.5, using 3N hydrochloric acid, and the solvents are removed *in vacuo* to give a residue, which is taken up in ethyl acetate and water (pH 2.5). Repeated extraction gives a combined ethyl acetate extract, which is dried (sodium sulfate), and evaporated to a residue. This residue is solubilized by addition of 5 ml of 0.5 M monobasic potassium phosphate buffer and adjusted to pH 6.5 using dilute aqueous potassium hydroxide. Chromatography on a column (120 ml) of HP—20 resin, using water as eluent gives 296 mg of the desired potassium salt. The lyophilized solid has a melting point 201°C, *dec.*

| | | | | |
|---|---|---|---|---|
| Anal. Calc'd for $C_{12}H_{15}N_6O_6S_2K{\cdot}0.5\ H_2O$: | C, 31.92; | H, 3.57; | N, 18.61; | S, 1420 |
| Found: | C, 32.08; | H, 3.62; | N, 18.51; | S, 14.07 |

Example 13

[3S-[3α(Z),4β]]-3-[[(2-Amino-4-thiazolyl)-[[2-(diphenylmethoxy)-1,1-dimethyl-2-oxoethoxy]imino]acetyl]amino]-4-methyl-N-(methylsulfonyl)-2-oxo-1-azetidinecarboxamide

[3S-[3α,4β]]-3-Amino-4-methyl-N-(methylsulfonyl)-2-oxo-1-azetidinecarboxamide trifluoroacetic acid salt (167 mg; see example 11) is dissolved in dry dimethylformamide (1.5 m,) and dry triethylamine (0.35 ml) and stirred at −25°C under dry nitrogen. To this solution is added, via syringe, a solution of mixed anhydride which is prepared by sequentially adding (Z)-2-amino-α-[[2-diphenylmethoxy)-1,1-dimethyl-2-oxoethoxy]imino]-4-thiazolecetic acid (220 mg), dry triethylamine (126 μl) and diphenylchlorophosphate (155 μl, .75 mmol) to dry dimethylformamide (1.5 ml) and stirring for 50 mionutes at −25C under nitrogen. The resulting mixture is stirred at −25°C for 2.5 hours under dry nitrogen. It is then poured into .5 M pH 5.5 monobasic potassium phosphate buffer (6 ml) and evaporated to dryness *in vacuo*. The residue is taken up in ethyl acetate/water, acidified with dilute hydrochloric acid to pH 2.5 and extracted (three times) with ethyl acetate. The extract is washed with water, dried over anhydrous sodium sulfate, evaporated to dryness *in vacuo*, and purified by thin-layer chromatography on silica gel in ethyl acetate:methanol (4:1). The eluted product is taken up in ethyl acetate/water at pH 2.5. The organic layer is washed with water, dried over anhydrous sodium sulfate, and evaporated to dryness *in vacuo* to give 152 mg of the desired product.

Example 14

[3S-[3α(Z),4β]]-3-[[(2-Amino-4-thiazolyl)-[(1-carboxy-1-methylethoxy)imino]acetyl]amino]-4-methyl-N-(methylsulfonyl)-2-oxo-1-azetidinecarboxamide, dipotassium salt

[3S - [3α(Z),4β]] - 3 - [[(2 - Amino - 4 - thiazolyl) - [[2 - (diphenylmethoxy) - 1,1 - dimethyl - 2 - oxoethoxy]imino]acetyl]amino] - 4 - methyl - N - (methylsulfonyl) - 2 - oxo - 1 - azetidinecarboxamide (152 mg; see example 13) is dissolved in dry dichloromethane. Anisole (135 μl) and trifluoroacetic acid (1 ml) are added, and the solution is stirred in an ice bath under dry nitrogen for 1.5 hours. The mixture is then evaporated to dryness *in vacuo* with the residue being evaporated *in vacuo* (four times) from dry acetonitrile to remove residual trifluoroacetic acid and anisole. The residue is taken up in ethyl acetate/water and 0.5 M pH 5.5 monobasic potassium phosphate buffer (2 ml). This is adjusted to pH 6.5 with dilute potassium hydroxide and extracted (two times) with ethyl acetate. The aqueous layer is evaporated to dryness *in vacuo*, and the residue is purified by chromatography on HP—20 resin (110 ml) using water. The product is lyophilized from water to give 82 mg, melting point 240°C, *dec.*

Anal. for $C_{15}H_{18}N_6O_8S_2K_2{\cdot}2\ H_2O$

| | | | | |
|---|---|---|---|---|
| Calc'd: | C, 30.60; | H, 3.77; | N, 14.28; | S, 10.89 |
| Found: | C, 30.61; | H, 3.43; | N, 14.28; | S, 10.51 |

Example 15

(S)-3-[[(1,1-Dimethylethoxy)carbonyl]amino]-N-(methylsulfonyl)-2-oxo-1-azetidinecarbothioamide

A) (S)-3-[[(1,1-Dimethylethoxy)carbonyl]amino]-2-oxoazetidine

(S)-3-Amino-2-azetidinone (21.77 g; see example 4A) and 30 g of triethylamine are dissolved in 200 ml of *t*-butanol and tetrahydrofuran (9:1). At 0°C a solution of 37 g of bis(*t*-butyl)pyrocarbonate in 50 ml of tetrahydrofuran is dropped in; stirring for about 16 hours completes the reaction. The solvents are distilled off and the residue is dissolved in ethyl acetate. Extraction with aqueous citric acid and water yields 25.3 g of the title compound from the organic phase.

B) (S(-3-[[(1,1-Dimethylethoxy)carbonyl]amino]-N-(methylsulfonyl)-2-oxo-1-azetidinecarbothioamide

Sec-Butyl lithium (9.25 ml of 1.35 M solution in cyclohexane) is added to a stirred solution of (S)-3-[[(1,1-dimethylethoxy)carbonyl]amino]-2-oxoazetidine (2.33 g) in 250 ml of dry tetrahydrofuran at −75°C under nitrogen. After stirring for 2 minutes, (methylsulfonyl)isothiocyanate (1.75 ml) is added, and the solution is stirred for 25 minutes at −75°C and poured into 125 ml of 0.5 M pH 5.5 monobasic potassium phosphate buffer. Ethyl acetate and water are added, and the pH is adjusted to 2.5 (3 N hydrochloric acid). The ethyl acetate layer is washed with water, dried with sodium sulfate, and evaporated to a residue (3.69 g), which is recrystallized from ethyl acetate to give 2.04 g of the title compound. Recrystallization of a portion from ethyl acetate gives crystals, melting point 181°C, *dec.*.

| | | | | |
|---|---|---|---|---|
| Anal. Calc'd for $C_{10}H_{17}N_3O_5S_2$: | C, 37.15; | H, 5.30; | N, 13.00; | S, 19.80 |
| Found: | C, 37.05; | H, 5.34; | N, 12.97; | S, 19.76 |

Example 16

(S)-3-Amino-N-(methylsulfonyl)-2-oxo-1-azetidinecarbothioamide, trifluoroacetic acid salt

(S) - 3 - [[(1,1 - Dimethylethoxy)carbonyl]amino] - N - (methylsulfonyl) - 2 - oxo - 1 - azetidinecarbothioamide (323 mg) is stirred with 3 ml of trifluoroacetatic acid at 0—5°C under nitrogen for 50 minutes. The solution is evaporated *in vacuo* to a residue, which is evaporated from acetonitrile (four times) to give the desired sale.

Example 17

[3S(Z)]-3-[[(2-Amino-4-thiazolyl)(methoxyimino)acetyl]amino]-N-(methylsulfonyl)-2-oxo-1-azetidinecarbothioamide, potassium salt

Triethylamine (162 µl) is added to a stirred suspension of (Z)-2-amino-α-(methoxyimino)-4-thiazole acetic acid (201 mg) in 3 ml of dry dimethylformamide at room temperature under nitrogen. After stirring for 2 minutes, the mixture is cooled to −25°C, and diphenyl chlorophosphate (208 µl), 1.0 mmol) is added. The mixture is stirred for 1.5 hours at −25°C and added, using a syringe, to a stirred solution of (S)-3-amino-N-(methylsulfonyl)-2-oxo-1-azetidinecarbothioamide, trifluoroacetic acid salt (1.0 mmol; see example 16) and 0.70 ml of triethylamine in 3 ml of dimethylformamide at −25°C under nitrogen. The reaction is stirred for 2.5 hours at this temperature and poured into 12 ml of 0.5 M pH 5.5 monobasic potassium phosphate buffer. Ethyl acetate and water are added, and the pH is adjusted to 7. The aqueous layer is adjusted to 2.5 (3 N hydrochloric acid), and the solvents are removed *in vacuo* to give a residue, which is taken up in ethyl acetate and water (pH 2.5). Repeated extraction with ethyl acetate gives a combined ethyl acetate extract, which is dried (sodium sulfate), and evaporated to a residue. This residue is solubilized by addition of 5 ml of 0.5 M monobasic potassium phosphate buffer. Adjustment of the pH to 6.5, using dilute potassium hydroxide, and chromatography on a column of HP20AG resin (110 ml), using water as eluant, yields 42 mg of the desired potassium salt as a residue. Treatment of a 36 mg portion of this sample with water and a small amount of acetone gives 31 mg of the desired potassium salt as crystals melting point 205°C, *dec.*.

| | | | | |
|---|---|---|---|---|
| Anal. Calc'd for $C_{11}H_{13}N_6O_5S_3K{\cdot}0.5\ H_2O$: | C, 29.14; | H, 3.11; | N, 18.54; | S, 21.17 |
| Found: | C, 29.40; | H, 3.05; | N, 18.52; | S, 21.13 |

Example 18

[3S(Z)]-3-[[(2-Amino-4-thiazolyl)(methoxyimino)acetyl]amino]-N-(aminosulfonyl)-2-oxo-1-azetidinecarboxamide, ammonium salt

[3S(Z)]-3-[[(2-(Triphenylmethylamino)-4-thiazolyl][methoxyimino]acetyl]amino]-2-oxo-azetidine (0.51 g; see example 4B) is suspended in 10 ml of anhydrous acetonitrile. The mixture is cooled to −30°C. A solution of chlorosulfonyl isocyanate (0.18 g) in 5 ml of acetonitrile is added with stirring. The mixture is stirred at −30°C for 30 minutes, the cooling bath is removed, and the mixture is stirred for an additional 60 minutes at 0°C. Ammonium carbonate (0.39 g) is added and the mixture is stirred at 0°C for 1 hour and at room temperature for an additional 2 hours. The precipitate is filtered, yielding 1.26 g of crude [3S(Z)]-3-[[(2-triphenylmethylamino)-4-thiazolyl](methoxyimino]acetyl]amino]-N-(aminosulfonyl)-2-oxo-1-azetidinecarboxamide, ammonium salt contaminated with some ammonium carbonate. The crude product is dissolved at room temperature in 12 ml of 70% formic acid and stirred for 90 minutes. The precipitate is filtered, the filtrate evaporated, the residue treated with water and adjusted to pH 6.5 with aqueous ammonia. The solution is filtered and freeze-dried. The freeze-dried product is purified by chromatography

on HP—20 resin, eluting with water. The first 100 ml are discarded, 10 ml fractions are collected. Fractions 25—31 contain 60 mg of pure product. From fractions 32—40 additional 30 mg of slightly less pure product are obtained.

Example 19

[3S(Z)]-3-[[(2-Amino-4-thiazolyl)(methoxyimino)acetyl]amino]-N-[[(1-methylethyl)amino]sulfonyl]-2-oxo-1-azetidinecarboxamide, sodium salt

[3S(Z)]-3-[[[2-(Triphenylmethylamino)-4-thiazolyl][methoxyimino]acetyl]amino]-2-azetidinone (1.02 g; see example 4B) is suspended in 10 ml of acetonitrile; the mixture is cooled to −30°C. A solution of chlorosulfonyl isocyanate (0.35 g) in 10 ml of acetonitrile is added with stirring. The mixture is stirred for 30 minutes at −30°C and for an additional 60 minutes at 0°C. The solution is cooled to −10°C and a solution of isopropylamine (0.5 g) in 5 mol of acetonitrile is added to form a clear solution. The cooling bath is removed and the mixture is stirred at 0°C for 60 minutes. After evaporation to dryness, 2.2 g of crude [3S(Z) - 3 - [[[2 - (triphenylmethylamino) - 4 - thiazolyl][methoxyimino]acetyl]amino] - N - [[(1 - methylethyl)-amino]sulfonyl] - 2 - oxo - 1 - azetidinecarboxamide as the isopropylamine salt is obtained as a light syrup. The syrup is dissolved in 35 ml of 70% formic acid and stirred for 3 hours at room temperature. A precipitate (0.48 g) is removed by filtration and the filtrate is evaporated to dryness. The residue is treated with water, adjusted to pH 6.5 with 2N sodium hydroxide, and freeze-dried. The crude product is subjected to HP20AG chromatography (100—200 mesh) eluting with water (500 ml), then with water/acetone (8:2); 10 ml fractions are taken. The elution is monitored by thin-layer chromatography. Fractions 72—76 contain most of the product and are divided into three parts: fractions 72—74 contain 0.19 g of product, fraction 75 contains 0.36 g of product and fraction 76 contains 0.16 g of product. The product from fractions 72—74 and 76 is slightly less pure than the product from fraction 75.

Example 20

(S)-N-(Aminosulfonyl)-2-oxo-3-[[(phenylmethoxy)carbonyl]amino]-1-azetidinecarboxamide

Method I

(S)-(2-Oxo-3-azetidinyl)carbamic acid, phenylmethyl ester (11 g; see example 1C) is dissolved in a mixture of 200 ml of acetonitrile and 50 ml of dichloromethane. The mixture is cooled to −50°C and a solution of chlorosulfonyl isocyanate (9 g) in 25 ml of dichloromethane is added with stirring. After warming the mixture to −30°C a solution of 6 g of ammonia in 160 ml of acetonitrile is added slowly. The reaction temperature is raised to −10°C and finally to 0—5°C. The reaction time is 3 hours. The ammonium salt of the title compound precipitates and is filtered by suction (20 g). The crude product is purified by HP—20 chromatography (100—200 mesh) eluting with 2000 ml of water and water/acetone (8:2); 20 ml fractions are taken. The elution is monitored by thin-layer chromatography. From fractions 143—154, 9.3 g of product is obtained by evaporation.

The ammonium salt of the title compound is dissolved in 100 ml of water, layered with 200 ml of ethyl acetate and acidified. After separation and washing of the aqueous layer twice with ethyl acetate, the organic layer is washed with saturated sodium chloride solution, dried with anhydrous magnesium sulfate and evaporated to yield 8.1 g of the title compound.

Method II

A mixture of (S)-(2-oxo-3-azetidinyl)carbamic acid, phenylmethyl ester (11 g; see example 1C) in 175 ml of dichloromethane is cooled to −30°C. While stirring 7.7 g of chlorosulfonyl isocyanate in 75 ml dichloromethane is added dropwise within 15 minutes. The temperature of the solution is allowed to rise to 0°C over 30 minutes. Subsequently the clear solution is again cooled to −30°C and 8.8 g of bis-(trimethylsilyl)amine dissolved in 30 ml of dichloromethane, is dropped in, while passing dry nitrogen through the flask. After an hour the reaction temperature is allowed to rise to −15°C and is maintained for an additional 30 minutes. The solvent is distilled off *in vacuo*, and the residue is triturated with 400 ml of ether to give a solid (16.6 g) which is washed with an additional 20 ml ether. From the ethereal mother liquor there is obtained a second crop of 4.2 g of product.

The crude material (18.0 g) along with about 20 g of HP—20 resin is suspended in 30 ml of water and the mixture is chromatographed on an HP—20 column eluted with a) 3 L of water; b) 2.5 L of water/acetone (8:2); c) 4 L of water/acetone (7:3); d) 6 L of water/acetone (6:4). Fraction d yields 6.2 g of the title compound melting point 150—152°C.

Example 21

(S)-N-(Aminosulfonyl)-3-[[(1,1-dimethylethoxy)carbonyl]amino]-2-oxo-1-azetidinecarboxamide, ammonium salt

(S)-3-[[(1,1-Dimethylethoxy)carbonyl]amino]-2-oxoazetidine (5.1 g; see example 15A) is dissolved in a mixture of 160 ml of acetonitrile and 40 ml of dichloromethane. At −50°C a solution of 5.4 g of chlorosulfonyl isocyanate in 30 ml of dichloromethane is added dropwise (15 minutes). The mixture is kept at −30°C for 30 minutes. Then 64 ml of a solution of ammonia in acetonitrile (containing 2.4 g of ammonia) is added slowly at −10 to 0°C. The title compound precipitates together with inorganic material and is filtered by suction (crude yield 9.8 g). From the filtrate another crop of 2.7 g of crude title compound is obtained. According to thin-layer chromatography both fractions are of similar purity. The combined material is

dissolved in 50 ml of water, adjusted to pH 6.5 by the addition of diluted aqueous ammonia and purified by chromatography on HP—20, eluting with water; 20 ml fractions are taken. Fractions 70—130 contain 2.7 g of the title compound.

Example 22

(S)-3-Amino-N-(aminosulfonyl)-2-oxo-1-azetidinecarboxamide, inner salt

Method I:

(S) - N - (Aminosulfonyl) - 3 - [[(1,1 - dimethylethoxy)carbonyl)amino] - 2 - oxo - 1 - azetidinecarboxamide, ammonium salt (2 g; see example 21) is added to 50 ml of trifluoroacetic acid at 0°C. The solution is stirred at 0°C for 10 minutes and then for 30 minutes at room temperature, evaporated to dryness and the residue is treated with ether to yield 2.1 g of the trifluoroacetic acid salt of the title compound, contaminated with ammonium trifluoroacetate. 1.6 g of the trifluoroacetic acid salt of the title compound is suspended in 25 ml of acetonitrile. Addition of 6 ml of bis-trimethylsilylacetamide yields a clear solution from which, on the addition of 5 ml methanol, the title compound (0.58 g) precipitates.

Method II:

(S)-3-[[(1,1-Dimethylethoxy)carbonyl]amino]-2-oxoazetidine (1.87 g; see example 15A) is dissolved in 50 ml of dichloromethane/acetonitrile (4:1) and cooled to −20°C. Chlorosulfonyl isocyanate (1.42 g) in 15 ml of dichloromethane is added dropwise. Stirring is continued for 3 hours and a solution of 1.9 g of N-trimethylsilyl *t*-butylcarbamate in 20 ml of dichloromethane is added and the mixture is stirred for 10 hours at 0°C. After this time the solvents are distilled off and the residue is treated with ether/petroleum ether (3:1) yielding 2 g of crude (S) - 3 - [[(1,1 - dimethylethoxy)carbonyl]amino] - N - [[(1,1 - dimethylethoxy)carbonyl]amino] - 2 - oxo - 1 - azetidinecarboxamide as a crystalline material. This is stirred for 90 minutes in 20 ml trifluoroacetic acid/anisole (4:1) at −10°C. The reaction solution is then poured into 200 ml of ether; 2.3 g of solid is collected and dried. Suspension of this solid in dry acetonitrile and addition of 3 g of monosilyltrifluoroacetamide yields a clear solution. To this solution 6 ml of methanol is added. The title compound precipitates from the solution immediately, yielding 0.76 g of product.

Example 23

[3S(Z)]-N-(Aminosulfonyl)-3-[[(2-amino-4-thiazolyl)(methoxyimino)acetyl]amino]-2-oxo-1-azetidinecarboxamide, sodium salt

(S)-3-Amino-N-(aminosulfonyl)-2-oxo-1-azetidinecarboxamide (see example 22, or prepared by hydrogenating 4.8 g of (S)-N-(aminosulfonyl)-3-[[(phenylmethoxy)carbonyl]amino]-2-oxo-1-azetidinecarboxamide using 2.5 g of 10% palladium on charcoal catalyst) is acylated using 4.22 g of (Z)-2-amino-α-(methoxyimino)-4-thiazoleacetic acid in 100 ml of dimethylformamide, 3.46 g of dicyclohexylcarbodiimide in 30 ml of dimethylformamide and 0.5 g of hydroxybenzotriazole. After 2.5 hours at room temperature the precipitated dicyclohexylurea is removed by filtration and the filtrate evaporated. The residue is triturated with 300 ml ethyl acetate to yield 4.2 g of insoluble material. After evaporation and trituration of the residue with ether another 1.6 g of solid are obtained. The combined solid material is suspended in water and the suspension adjusted to pH 6.5 with 1N sodium hydroxide, filtered and the filtrate subjected to HP—20 chromatography, eluting with water. The first 700 ml of eluate are discarded. Fractions 85—137 contain 1.04 g of the title compound m.p. 230°C. (dec.). Another 0.3 g of slightly less pure material are obtained from fractions 70—84 and 138—150.

Example 24

(S)-N-(Aminosulfonyl)-3-[[(phenylmethyl)carbonyl]amino]-2-oxo-1-azetidinecarboxamide

(S)-3-Amino-N-(aminosulfonyl)-2-oxo-1-azetidinecarboxamide (see example 22), or prepared by hydrogenating 140 mg of (S)-N-(aminosulfonyl)-3-[[(phenylmethoxy)carbonyl]amino]-2-oxo-1-azetidinecarboxamide in dimethylformamide using 50 mg of 5% palladium on charcoal catalyst) is acylated with 100 mg of dicyclohexylcarbodiimide, 20 mg of hydroxybenzotriazole and 55 mg of benzeneacetic acid. The mixture is stirred at 0°C for 6 hours and dicyclohexylurea is filtered off. Dimethylformamide is distilled off, the residue is dissolved in 5 ml of acetone and the solution is, after standing for about 16 hours, filtered and then evaporated. The crude material is chromatographed on an HP—20 column eluting with water/acetone (6:4) to give 45 mg of the title compound, melting point 137°C.

Example 25

(S)-3-[[Phenylmethoxy)carbonyl]amino]-N-[[(phenylmethoxy)amino]sulfonyl]-2-oxo-1-azetidinecarboxamide, potassium salt

(S)-(2-Oxo-3-azetidinyl)carbamic acid, phenylmethyl ester (5.1 g; see example 1C) is suspended in 80 ml of dry dichloromethane at −50°C. At this temperature 3.9 g chlorosulfonyl isocyanate dissolved in 25 ml of dichloromethane is dropped in with stirring. When the addition is complete, a clear solution is obtained and stirring is continued for 2 hours. Triethylamine (5.6 g) is added and 3.7 g of benzyloxyamine, dissolved in 20 ml of dichloromethane is added dropwise to the solution. The reaction mixture is stirred for about 16 hours at 0°C. Tetrabutylammoniumhydrogensulfate (8.5 g) dissolved in 150 ml of ice water is added and the pH is adjusted to 6.7 with 1N potassium hydroxide. The organic layer is separated, dried with sodium

sulfate, and the solvent removed *in vacuo*. The oily residue is treated with 8.5 g of potassium perfluorobutanesulfonate in 50 ml of acetone. This solution is slowly poured into 200 ml of ether; crude product precipitates and is filtered off. Purification is achieved by reverse-phase chromatography using HP—20 and water/acetone 6:4 as eluent, yielding 3.2 g of product, melting point 200°C, *dec.*

Example 26

[3S(Z)]-3-[[(2-Amino-4-thiazolyl)(methoxyimino)carbonyl]amino]-N-[[(phenylmethoxy)amino]sulfonyl]-2-oxo-1-azetidinecarboxamide, potassium salt

(S) - 3 - [[Phenylmethoxy)carbonyl]amino] - N - [[(phenylmethoxy)amino]sulfonyl] - 2 - oxo - 1 - azetidinecarboxamide, potassium salt (1.2; see example 25) is dissolved in 80 ml of dry dimethylformamide and hydrogenated in the presence of 0.75 g of palladium on charcoal. After 40 minutes the hydrogenation is complete. The catalyst is filtered off, and 0.55 g of (Z)-2-amino-α-(methoxyimino)-4-thiazoleacetic acid, 0.17 g of hydroxybenzotriazole and 1.03 g of dicyclohexylcarbodiimide are added. The solution is stirred at ambient temperature for about 16 hours. The precipitated urea is filtered off, the solvent removed *in vacuo* and the residue is chromatographed using HP—20 and water/acetone (9:1) as eluant. The product is a mixture of 50 mg of the title compound and 200 mg of [3S(Z)]-3-[[(2-amino-4-thiazolyl)(methoxyimino)carbonyl]amino]-N-(aminosulfonyl)-2-oxo-1-azetidinecarboxamide, potassium salt.

Example 27

[3S(Z)]-3-[[(2-Amino-4-thiazolyl)(ethoxyimino)acetyl]amino]-2-oxo-1-azetidinecarboxamide, potassium salt

(S)-N-(Aminosulfonyl)-2-oxo-3-[[(phenylmethoxy)carbonyl]amino]-1-azetidinecarboxamide, potassium salt (0.5 g; see example 20) is hydrogenated for 20 minutes in 70 ml of dry dimethylformamide with 0.2 g of palladium on charcoal. (Z)-2-Amino-α-(ethoxyimino)-4-thiazoleacetic acid (0.36 g), 0.1 g of hydroxybenzotriazole and 0.62 g of dicyclohexylcarbodiimide are added and the solution is stirred for about 16 hours. The precipitated urea is filtered off, the solvent is removed and the residue is suspended in 5 ml of water. The pH is adjusted to 6.5 with 1N potassium hydroxide. This solution is immediately chromatographed using HP—20 and water as eluent, yielding 200 mg of the title compound, melting point 198—200°C, *dec.*

Example 28

[3S(R*)]-N-(Aminosulfonyl)-3-[[[[[3-[(2-furanylmethylene)amino]-2-oxo-1-imidazolidinyl]carbonyl]amino]phenylacetyl]amino]-2-oxo-1-azetidinecarboxamide, potassium salt

(S)-N-(Aminosulfonyl)-2-oxo-3-[[(phenylmethoxy)carbonyl]amino]-1-azetidinecarboxamide (0.8 g; see example 20) is dissolved in 80 ml of dry dimethylformamide and hydrogenated with 0.4 g of palladium on charcoal. After 20 minutes the catalyst is filtered off and 0.92 g of (R)-α-[[[3-[(2-furanylmethylene)amino]-2-oxo-1-imidazolidinyl]carbonyl]amino]benzeneacetic acid, 0.16 g of hydroxybenzotriazole and 0.97 g of dicyclohexylcarbodiimide are added and the solution is stirred at ambient temperature for about 16 hours. The precipitated urea is filtered off and the solvent is removed *in vacuo*. The residue is chromatographed on HP—20 using water/acetone (7:3) as eluent. Freeze-drying of the appropriate fractions yields 200 mg of product. The potassium salt is prepared by dissolving the compound in water/acetone and adjusting the pH to 6.7 with 1N potassium hydroxide. The solution is freeze-dried.

Example 29

[3S(R*)]-N-(Aminosulfonyl)-3-[[[[(4-ethyl-2,3-dioxo-1-piperazinyl)carbonyl]amino]phenylacetyl]amino]-2-oxo-1-azetidinecarboxamide

(S)-N-(Aminosulfonyl)-2-oxo-3-[[(phenylmethoxy)carbonyl]amino]-1-azetidinecarboxamide (0.8 g; see example 20) is hydrogenated in the presence of 0.4 g of palladium on charcoal. After 20 minutes the catalyst is filtered off and 0.82 g of (R)-α-[[(4-ethyl-2,3-dioxo-1-piperazinyl)carbonyl]amino]benzeneacetic acid, 0.16 g of hydroxybenzotriazole and 0.97 g of dicyclohexylcarbodiimide are added. The solution is stirred about 16 hours at ambient temperature. The precipitated urea is filtered off and the solvent is removed *in vacuo*. The residue is suspended in 10 ml of water/acetone and the pH is adjusted to 6.5 with 1N potassium hydroxide, and then immediately chromatographed (HP—20, water/acetone: 9/1) to yield 330 mg of product, m.p. $K^+$ salt 185°C (dec.).

Example 30

[3S(Z)]-2-[[[1-(2-Amino-4-thiazolyl)-2-[[1-[[(methylsulfonyl)amino]carbonyl]-2-oxo-3-azetidinyl]amino]-2-oxoethylidene]amino]oxy]-2-methylpropanoic acid, dipotassium salt

A) (S)-3-[[(1,1-Dimethylethoxy)carbonyl]amino]-N-(methylsulfonyl)-2-oxo-1-azetidinecarboxamide

(S)-3-[[(1,1-Dimethylethoxy)carbonyl]amino]-2-azetidinone (1.12 g) is dissolved in dry tetrahydrofuran (120 ml) and stirred at −75°C under dry nitrogen. sec-Butyl lithium (4.44 ml of a 1.35 M solution in cyclohexane) is added and followed in two minutes by methylsulfonyl isocyanate (0.72 ml). After stirring for 25 minutes at −75°C, the reaction is quenched by the addition of 0.5 M pH 5.5 monobasic potassium phosphate buffer (60 ml), diluted with ethyl acetate-water, acidified to pH 2.5 with 3N hydrochloric acid, and (60 ml), diluted with ethyl acetate-water, acidified to pH 2.5 with 3N hydrochloric acid, and extracted with ethyl acetate (three times). The extract is washed with water, dried over anhydrous sodium sulfate, and evaporated to dryness *in vacuo* to yield the desired product as a foam (1.73 g).

B) (S)-3-Amino-N-(methylsulfonyl)-2-oxo-1-azetidinecarboxamide, trifluoroacetic acid salt

(S)-3-[[(1,1-Dimethylethoxy)carbonyl]amino]-N-(methylsulfonyl)-2-oxo-1-azetidinecarboxamide (612 mg) is stirred with trifluoroacetic acid (6 ml) under nitrogen in an ice bath for 30 minutes. The solvent is removed *in vacuo*, and the residue is evaporated from acetonitrile (four times) to give the desired salt as a foam (638 mg).

C) [3S(Z)]-2-[[[1-(2-Amino-4-thiazolyl)-2-[[1-[[(methylsulfonyl)amino]carbonyl]-2-oxo-3-azetidinyl]amino]-2-oxoethylidene]amino]oxy]-2-methylpropanoic acid, diphenylmethyl ester

(Z)-(2-Amino-4-thiazolyl)[[2-(diphenylmethoxy)-1,1-dimethyl-2-oxoethoxy]imino]acetic acid (880 mg) in dry dimethylformamide (6 ml) is stirred at −25°C under nitrogen. Triethylamine (0.48 ml) is added, followed by diphenylchlorophosphate (0.62 ml), and the mixture is stirred at −25°C for 55 minutes. This mixture is added to a stirred solution of the above mentioned crude trifluoroacetic acid salt (612 mg), and triethylamine (1.40 ml) in dry dimethylformamide (6 ml) at −25°C under nitrogen. The reaction is stirred at 25°C for 2.5 hours and quenched with 0.5 M pH 5.5 monobasic potassium phosphate buffer (24 ml). It is then diluted with ethyl acetate/water, acidified to pH 2.5, and extracted with ethyl acetate (three times). The ethyl acetate extract is washed with water, dired over anhydrous sodium sulfate, and evaporated to dryness *in vacuo* to yield a yellow solid (1.408 g). After chromatography on silica gel, using ethyl acetate-methanol, the product is taken up in ethyl acetate-water at pH 2.5 and extracted with ethyl acetate (three times). The extract is washed with water, dried over anhydrous sodium sulfate, and evaporated to dryness *in vacuo* to give the desired product as a solid (360 mg).

D) [3S(Z)]-2-[[[1-(2-Amino-4-thiazolyl)-2-[[1-[[(methylsulfonyl)amino]carbonyl]-2-oxo-3-azetidinyl]amino]-2-oxoethylidene]amino]oxy]-2-methylpropanoic acid, dipotassium salt

[3S(Z)] - 2 - [[[1 - (2 - Amino - 4 - thiazolyl) - 2 - [[1 - [[(methylsulfonyl)amino]carbonyl] - 2 - oxo - 3 - azetidinyl]amino] - 2 - oxoethylidene]amino]oxy] - 2 - methylpropanoic acid, diphenylmethyl ester (360 mg) is dissolved in dry dichloromethane (10 ml) and stirred under dry nitrogen in an ice bath. Anisole (312 µl) and anhydrous trifluoroacetic acid (2 ml) are added, and the solution is stirred for 2 hours. The solvent is then removed *in vacuo*, and the residual solid is evaporated (four times) *in vacuo* from a solution in dry acetonitrile to remove traces of trifluoroacetic acid. The product is taken up in ethyl acetate-water. 0.5 M pH 5.5 monobasic potassium phosphate buffer (2 ml) is added, and the pH is adjusted to 6.5 with dilute potassium hydroxide. The organic layer is removed, and the aqueous layer is evaporated to a residue, which is combined with a similar residue, derived by treating a second portion (176 mg) of diphenylmethyl ester with trifluoroacetic acid-anisole. This crude potassium salt mixture is purified by chromatography on HP20AG, using water, to give the desired dipotassium salt as a powder (221 mg) after lyophilization, melting point 250—255°C, *dec.*

Analysis for $C_{14}H_{16}O_8N_6S_2 \cdot K_2 \cdot 3H_2O$

| | | | | |
|---|---|---|---|---|
| Calc'd: | C, 28.37; | H, 3.74; | N, 14.18; | S, 10.82 |
| Found: | C, 28.38; | H, 3.58; | N, 14.05; | S, 10.82 |

Example 31

(S)-N-(1-Imidazolylsulfonyl)-2-oxo-3-[[(phenylmethoxy)carbonyl]amino]-1-azetidinecarboxamide, potassium salt

(S)-(2-Oxo-3-azetidinyl)carbamic acid, phenylmethyl ester (3 g) is suspended in 100 ml of dry dichloromethane and cooled to −5°C. Chlorosulfonyl isocyanate (2.1 g) in 10 ml of dichloromethane is dropped in with stirring, which is continued for 45 minutes. Triethylamine (3.5 g) and 1.02 g of imidazole are added and the solution is stirred overnight at 0°C. After the addition of 4.6 g of tetrabutylammonium hydrogen sulfate in 200 ml of ice water, the pH is adjusted to 6.5 with 1N potassium hydroxide. The organic layer is separated, dried (sodium sulfate), filtered and evaporated to dryness. The residue is treated with 4.6 g of potassium perfluorobutanesulfonate in 50 ml of acetone. This solution is poured into 200 ml of ether and crude product (4.5 g) is filtered off. Purification is accomplished by chromatography using HP20AG resin and water/acetone (8:2) as eluant, yielding 1.7 g of the title compound, melting point 130°C, dec.

Example 32

[3S(Z)]-3-[[(2-Amino-4-thiazolyl)(methoxyimino)carbonyl]amino]-N-(1-imidazolylsulfonyl)-2-oxo-1-azetidinecarboxamide, potassium salt

(S)-N-(1-Imidazolylsulfonyl)-2-oxo-3-[[(phenylmethoxy)carbonyl]amino]-1-azetidinecarboxamide, potassium salt (0.5 g; see example 31) is dissolved in 50 ml of dry dimethylformamide. After the addition of 0.2 g of 10% palladium on charcoal, hydrogen is bubbled through the mixture for 20 minutes. The catalyst is filtered off and 0.23 g of (Z)-2-amino-α-(methoxyimino)-4-thiazoleacetic acid. 0.05 g of hydroxybenzotriazole and 0.5 g of dicyclohexylcarbodiimide are added. The solution is stirred for 12 hours at room temperature. The precipitated urea is filtered off and the solvent is removed *in vacuo*. The residue is purified by HP20 chromatography using water/acetone (9:1), yielding 0.3 g of the title compound, melting point 210°C, *dec.*

Example 33

[3S(Z)]-2-[[[1-(2-Amino-4-thiazolyl)-2-[[1-[[(aminosulfonyl)amino]carbonyl]-2-oxo-3-azetidinyl]amino]-2-oxoethylidene]amino]oxy]-2-methylpropanoic acid, disodium salt

(S)-3-Amino-N-(aminosulfonyl)-2-oxo-1-azetidinecarboxamide, inner salt (see example 22) is reacted with 3.2 g (a 20% excess) of (Z)-(2-amino-4-thiazolyl)[[2-(diphenylmethoxy)-1,1-dimethyl-2-oxoethoxy]-imino]acetic acid in the presence of 1.5 g of dicyclohexylcarbodiimide and 0.2 g of N-hydroxybenzotriazole. The mixture is stirred for 5 hours at room temperature, the solution is evaporated and the residue is dissolved in ethyl acetate. The insoluble dicyclohexylurea is filtered off and the filtrate is washed three times with water and once with saturated sodium chloride solution, dried (magnesium sulfate) and evaporated to yield 4 g of crude [3S(Z)] - 2 - [[[1 - (2 - amino - 4 - thiazolyl) - 2 - [[1 - [[(aminosulfonyl)amino]-carbonyl] - 2 - oxo - 3 - azetidinyl]amino] - 2 - oxoethylidene]amino]oxy] - 2 - methylpropanoic acid, diphenylmethyl ester.

A portion of the crude is dissolved in 15 ml of anisole, cooled with stirring to −10°C and 40 ml of trifluoroacetic acid is added. The mixture is stirred at −10°C for an additional 30 minutes. Then, at −10°C, 100 ml of ether and 100 ml of petroleum ether are added to yield a precipitate (2.4 g). The precipitate is suspended in 50 ml of water at 0°C and adjusted to pH 6.5 by the slow addition of 2N sodium hydroxide. Some insoluble material is removed by filtration and the filtrate freeze-dried to yield 2 g of crude product. The crude material is purified by HP20AG chromatography eluting with water; 10 ml fractions are taken. From fractions 22—26, 0.63 g of pure product are obtained after freeze-drying, m.p. 275°C (dec.) as disodium salt. From fractions 20—21 and 27—29 another 0.39 g of less pure product are obtained.

Example 34

[3S(Z)]-3-[[(2-Amino-4-thiazolyl)(methoxyimino)acetyl]amino]-2-oxo-N-[(phenylamino)sulfonyl]-1-azetidinecarboxamide, sodium salt

[3S(Z)] - 3 - [[[2 - (Triphenylmethylamino) - 4 - thiazolyl][methoxyimino]acetyl]amino] - 2 - oxoazetidine (1.02 g; see example 4B) is suspended in a mixture of 13 ml of acetonitrile and 13 ml of dichloromethane. At −50°C a solution of chlorosulfonyl isocyanate (0.36 g) in 5 ml of dichloromethane is dropped in with stirring. The temperature is raised to −20°C within 30 minutes. Addition of triethylamine (0.84 ml) immediately yields a clear solution to which a solution of aniline (0.22 ml) is added. The reaction mixture is stirred at 0°C for 60 minutes and at room temperature for another 60 minutes, then evaporated. The residue is suspended in water and the suspension is adjusted to pH 3.5 by the addition of 2N acetic acid. The insoluble crude [3S(Z)]-3-[[[(triphenylmethyl)amino]-4-thiazolyl][methoxyimino]acetyl]amino]-2-oxo-N-[(phenylamino)sulfonyl]-1-azetidinecarboxamide is isolated by filtration, yield 1.2.

To remove the trityl group, 1.1 g of (3S(Z)]-3-[[[2-(triphenylmethylamino)-4-thiazolyl][methoxyimino]-acetyl]amino]-2-oxo-N-[(phenylamino)sulfonyl]-1-acetidinecarboxamide is dissolved in 15 ml of tetrahydrofuran and 25 ml of 70% formic acid is added with cooling. After standing for 2 hours at room temperature the mixture is evaporated and the residue is treated with ether to yield 0.59 g of solid material. The solid is dissolved in 10 ml of tetrahydrofuran. Then 10 ml of water is added and the mixture is adjusted to pH 6.5 by the addition of 2N sodium hydroxide. The tetrahydrofuran is removed by evaporation, the turbid water-phase is extracted once with ethyl acetate and freeze-dried. The crude material thus obtained is purified by HP20AG chromatography; 10 ml fractions are collected. It is first eluted with water (fractions 1—80), then with water/acetone (95:5). From fractions 98—108, 70 mg of pure product is obtained.

Example 35

[3S(Z)]-3-[[(2-Amino-4-thiazolyl)(methoxyimino)acetyl]amino]-N-[(dimethylamino)sulfonyl]-2-oxo-1-azetidinecarboxamide, sodium salt

Following the procedure of example 34, but substituting dimethylamine for aniline, yields the title compound.

Example 36

(S)-2-Oxo-3-[[(phenylmethoxy)carbonyl]amino]-N-[[(4-pyridyl)amino]sulfonyl]-1-azetidinecarboxamide, sodium salt

(S)-(2-Oxo-3-azetidinyl)carbamic acid, phenylmethyl ester (6.6 g; see example 1C) is suspended in a mixture of 160 ml of acetonitrile and 40 ml of dichloromethane. To the suspension a solution of chlorosulfonyl isocyanate (5.1 g) in 15 ml dichloromethane is added at −50°C with stirring. The mixture is stirred for 1 hour at −30 to −25°C. A clear solution of (S)-N-[(chlorosulfonyl)amino]-2-oxo-3-[[(phenylmethoxy)carbonyl]amino]-1-azetidinecarboxamide forms to which 12.4 ml of triethylamine is added, followed by a suspension of (4-pyridyl)amine (3.4 g) in 40 ml of a 1:1 mixture of acetonitrile and dichloromethane. The temperature is raised to 0—5°C and the mixture is stirred at this temperature for 90 minutes. A clear solution forms, from which crystals precipitate. After stirring for an additional 2 hours at room temperature the precipitate is filtered by suction, suspended in water and the pH of the suspension is adjusted to pH 3.5. After stirring for 30 minutes, the precipitate is filtered, washed with water and dried to yield 8 g of the title compound, m.p. 212°C. From the organic filtrate a second crop of 3.4 g of less pure product is obtained by evaporation, suspension in water, adjustment of the suspension to pH 3.5 and filtration.

Example 37

(S)-N-[(Methylamino)sulfonyl]-2-oxo-3-[[(phenylmethoxy)carbonyl]amino]-1-azetidinecarboxamide

(S)-(2-Oxo-3-azetidinyl)carbamic acid, phenylmethyl ester (6.6 g; see example 1C) is suspended in a mixture of 160 ml of acetonitrile and 40 ml of dichloromethane. Then, at −50°C, chlorosulfonyl isocyanate (5.1 g) dissolved in 15 ml of dichloromethane, is added with stirring. After stirring for 1 hour at −30°C, a solution of 6 g of methylamine in 50 ml of acetonitrile is added so that an excess of amine is avoided (pH ca. 6.5) (40 ml of the solution is required). After stirring for 90 minutes at −10°C and 90 minutes at 0°C the precipitate is filtered to yield 10.5 g of a solid. Evaporation of the filtrate yields another 5.2 g of a foam. According to the spectra (NMR, IR) both contain the methylammonium salt of the title compound. The combined material is purified by chromatography on HP20AG. The methylammonium salt of the title compound is eluted with water/acetone (80:20) to yield 4.6 g of pure material. From the methylammonium salt, the title compound is prepared by dissolving it in water (50 ml), layering with ethyl acetate and acidifying the mixture with cooling and stirring. From the organic layer 3.5 g of the title compound is obtained.

Example 38

(S)-3-Amino-N-[(methylamino)sulfonyl]-2-oxo-1-azetidinecarboxamide, inner salt

(S) - N - [(Methylamino)sulfonyl] - 2 - oxo - 3 - [[(phenylmethoxy)carbonyl]amino] - 1 - azetidinecarboxamide (2.1 g; see example 37) is suspended in 25 ml of acetonitrile and bis(trimethylsilyl)acetamide (3.3 g) is added. The solution is added to a suspension of 1 g of 10% palladium on charcoal in 25 ml of acetonitrile, through which hydrogen is passed for 30 minutes prior to the addition of the solution of the starting azetidinone. After 15 minutes the catalyst is removed by filtration and 2 ml of methanol is added to the filtrate to precipitate 1.3 g of the title compound.

Example 39

(S)-3-Amino-2-oxo-N-[[(4-pyridyl)amino]sulfonyl]-1-azetidinecarboxamide

(S) - 2 - Oxo - 3 - [[(phenylmethoxy)carbonyl]amino] - N - [[(4 - pyridyl)amino]sulfonyl] - 1 - azetidinecarboxamide (2.1 g; see example 36) is suspended in 25 ml of acetonitrile and 3 g (3.6 ml) of bis(trimethylsilyl)acetamide is added with stirring. After a few minutes a clear solution is formed. This solution is added to a suspension of 1 g of 10% palladium on charcoal in 25 ml of acetonitrile, through which hydrogen is passed for 30 minutes prior to the addition of the solution of the starting azetidinone. Hydrogenation is complete after 70 minutes. The catalyst is removed by filtration. On addition of 1 ml of methanol the title compound crystallizes from the solution; yield 1.0 g.

Example 40

[3S(Z)]-3-[[(2-Amino-4-thiazolyl)(methoxyimino)acetyl]amino]-2-oxo-N-[[(4-pyridyl)amino]sulfonyl]-1-azetidinecarboxamide

(S)-3-Amino-2-oxo-N-[[(4-pyridyl)amino]sulfonyl]-1-azetidinecarboxamide (0.57 g; see example 39) is dissolved in 10 ml of dimethylformamide and added to a mixture of (Z)-2-amino-α-(methoxyimino)-4-thiazoleacetic acid (0.6 g), dicyclohexylcarbodiimide (0.49 g) and N-hydroxybenzotriazole (60 mg) in 20 ml of dimethylformamide and stirred at room temperature. After 2 hours the conversion is complete. Dicyclohexylurea is removed by filtration and the filtrate is evaporated and treated with ether to yield 1.3 g of a solid. The solid is dissolved in a mixture of 15 ml of water and 15 ml of acetone and adjusted to pH 6.5 by the addition of a solution of sodium bicarbonate. On removal of the acetone by evaporation, 0.5 g of the title compound precipitates from the aqueous phase, m.p. 221°C. (dec.).

Example 41

[3S(Z)]-2-Amino-N-[1-[[[(dimethylamino)sulfonyl]amino]thioxomethyl]-2-oxo-3-azetidinyl]-α-(methoxyimino)-4-thiazoleacetamide

A) (S)-3-[[(1,1-Dimethylethoxy)carbonyl]amino]-N-[[[1-(dimethylamino)sulfonyl]amino]thioxomethyl]-2-oxoazetidine

(S)-3-[[(1,1-Dimethylethoxy)carbonyl]amino-2-oxoazetidine (0.93 g, .005 mole) and triethylamine (1.35 ml, .01 mole) were dissolved in 20 ml of dry acetonitrile at 0°C. [Dimethylamino(sulfonyl)]isothiocyanate (1.09 ml, .01 mole) was added dropwise and the reaction was stirred at 0°C for 3 hrs and at room temperature overnight. The reaction mixture was concentrated *in vacuo*. To the residue was added 10 ml of 0.5N pH 5.5 $KH_2PO_4$ and 5 ml of water. The pH was adjusted to 2.8 with dilute acid (1N HCl), and product was extracted with 3 200 ml portions of ethyl acetate. The ethyl acetate was concentrated *in vacuo* and product was recrystallized from methanol to yield 0.76 g, m.p. dec. 184°C.

B) (S)-3-Amino-N-[[[(dimethylamino)sulfonyl]amino]thioxomethyl]-2-oxoazetidine, trifluoroacetic acid salt

The above thioxomethyl azetidine (0.352 g, .001 mole) was dissolved in 3 ml of trifluoroacetic acid and stirred at 0° for 2 hrs. The solution was concentrated *in vacuo*, and the residue was evaporated from acetonitrile (3×) to give the desired product.

C) [3S(Z)]-2-Amino-N-[1-[[[(dimethylamino)sulfonyl]amino]thioxomethyl]-2-oxo-3-azetidinyl]-α-(methoxyimino)-4-thiazoleacetamide, potassium salt

To a solution of (2-amino-4-thiazolyl)methoxyimino acetic acid (0.266 g, .001 mole) in 3 ml dry DMF was added triethylamine (.153 ml, .0011 mole) under $N_2$. This solution was cooled to −25°C and diphenyl chlorophosphate (0.207 ml, .001 mole) was added. The reaction mixture was stirred at −25°C for 1 hr. The above crude trifluoroacetic acid salt and triethylamine (0.7 ml) was dissolved in 3 ml of DMF. The above mixed anhydride was added via syringe to this solution at −50°C to −25°C. After 3 hrs. the solution was poured into 12 ml of 0.5M pH 5.5 $KH_2PO_4$ buffer, and the pH was adjusted to 7.8 with dilute KOH. The DMF and water were evaporated off *in vacuo* and the pot residue was dissolved in 20 ml of water and washed with ethyl acetate. The aqueous solution was acidified to pH 2.8 and product was extracted with two portions of ethyl acetate. This was dried ($Na_2SO_4$), filtered, and concentrated *in vacuo*. The product was purified by chromatographing twice through two 60 ml portions of HP—20 resin using water as eluant. The aqueous fractions containing product (Rydon positive) were lyophilized to give 109 mg of analytical product, after drying at 45°C/1 mm for 4 hrs, having m.p. 204—208° dec.

Example 42

[3S(R)]-4-Ethyl-N-]2-[[1-[[(methylsulfonyl)amino]carbonyl]-2-oxo-3-azetidinyl]amino]-2-oxo-1-phenylethyl]-2,3-dioxo-1-piperazinecarboxamide

By substituting (R)-α-[[(4-ethyl-2,3-dioxo-1-piperazinyl)carbonyl]amino]phenyl acetyl chloride for the phenylacetyl chloride used in Example 7, the titled product is obtained as an amorphous solid, m.p. 181—185°C as the sodium salt.

Example 43

[2S-[2α,3β(R)]]-4-Ethyl-N-[2-[[2-methyl-1-[[(methylsulfonyl)amino]carbonyl]-4-oxo-3-azetidinyl]amino]-2-oxo-1-phenylethyl]-2,3-dioxo-1-piperazinecarboxamide

By reacting (R)-α-[[4-ethyl-2,3-dioxo-1-piperazinyl)carbonyl]amino]phenyl acetic acid and [3S-[3α,4β]]-3-amino-4-methyl-N-(methylsulfonyl)-2-oxo-1-azetidinecarboxamide (from Example 11) according to the procedure of Example 7, the titled product is obtained as a solid, m.p. 170°C. (d), as the sodium salt.

Example 44

[3S(R)]-3-[(Aminophenylacetyl)amino]-N-(methylsulfonyl)-2-oxo-1-azetidinecarboxamide

By substituting α-amino-phenyl acetic acid for the (2-amino-4-thiazolyl)-(methoxyimino)acetic acid used in Example 12, the titled product is obtained, m.p. 149°C. (d.) as the trifluoroacetic acid salt.

Example 45

[3S(Z)]-2-Amino-N-[1-[[[(4-aminophenyl)sulfonyl]amino]carbonyl]-2-oxo-3-azetidinyl]-α-(methoxyimino)-4-thiazoleacetamide

By substituting (S)-3-amino-N-[(4-aminophenyl)sulfonyl]-2-oxo-1-azetidinecarboxamide for the azetidinecarboxamide used in Example 12, the titled product is obtained as a solid (potassium salt) m.p. 220°C. (d.).

Example 46

[3S(Z)]-2-[[[1-(2-Amino-4-thiazolyl)-2-[[1-[[(1H-imidazol-1-ylsulfonyl)amino]carbonyl]-2-oxo-3-azetidinyl]amino]-2-oxoethylidene]-amino]oxy]-2-methylpropanoic acid

By following the procedure of Example 33, substituting (S)-N-(1-imidazolylsulfonyl)-2-oxo-1-azetidinecarboxamide for the azetidinecarboxamide used therein, the titled product is obtained, m.p. potassium salt 240°C. (d.).

Example 47

[3S(R)]-4-Ethyl-N-[2-[[1-[[(1H-imidazol-1-ylsulfonyl)amino]carbonyl]-2-oxo-3-azetidinyl]amino]-2-oxo-1-phenylethyl]-2,3-dioxo-1-piperazinecarboxamide

By reacting (S)-N-(1-imidazolylsulfonyl)-2-oxo-1-azetidinecarboxamide and (R)-α-[[-4-ethyl-2,3-dioxo-1-piperazinyl)carbonyl]amino]phenylacetic acid in the presence of dicyclohexylcarbodiimide and N-hydroxybenzotriazole according to the procedure of Example 33, the titled product is obtained, potassium salt m.p. 175°C. (d.).

Example 48

[3S(Z)-2-amino-N-[1-[[[thiazol-2-ylamino)sulfonyl]amino]thioxomethyl]-2-oxo-3-azetidinyl]-α-(methoxyimino)-4-thiazoleacetamide

By substituting (S)-3-amino-2-oxo-N-[[(2-thiazolinyl)amino]sulfonyl]-1-azetidinecarboxamide for the carboxamide used in Example 40, the titled compound is obtained, m.p. sodium salt, 250°C (d.).

Example 49

[3S(Z)-2-amino-N-[1-[[[5-methyl-1,3,4-thiadiazol-2-ylamino]sulfonyl]amino]thioxomethyl]-2-oxo-3-azetidinyl]-α-(methoxyimino)-4-thiazoleacetamide

By substituting (S)-3-amino-2-N-[[(5-methyl-1,3,4-thiadiazole-2-yl)amino]sulfonyl]-1-azetidinecarboxamide for the carboxamide used in Example 40, the titled compound is obtained, m.p. 209—212°C.

Example 50

By substituting the carboxylic acids containing the acyl groups shown in the first column of Table I into Example 23 for the acid used therein (2-amino-α-(methoxyimino)-4-thiazoleacetic acid), and in the case of parts (j) through (o), substituting the azetidinecarboxamide containing the R-radical indicated, for the azetidinecarboxamide used in Example 23, the products shown in Table I are obtained.

TABLE 1

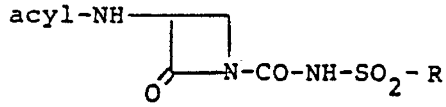

| | Acyl | –R | m.p. °C. |
|---|---|---|---|
| (a) | 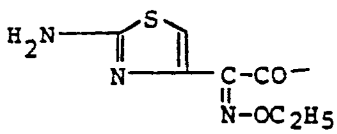 | $-NH_2$ | 200 d. $K^+$ salt |
| (b) | 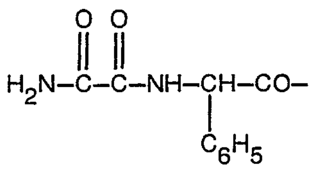 | $-NH_2$ | 212 d. $K^+$ salt |
| (c) | 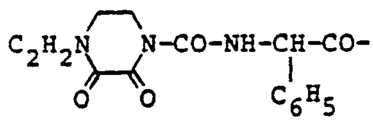 | $-NH_2$ | 185 d. $K^+$ salt |
| (d) | 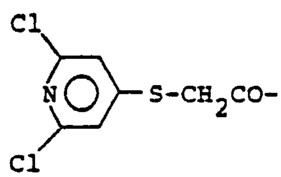 | $-NH_2$ | 100–105° |
| (e) | 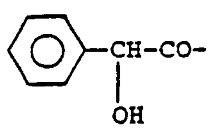 | $-NH_2$ | 132–135° d. |
| (f) | 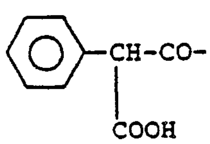 | $-NH_2$ | 145° d. |
| (g) | 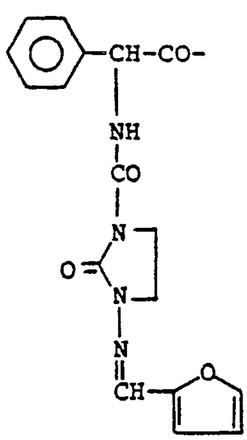 | $-NH_2$ | 245° d. $K^+$ salt |

TABLE 1 (Continued)

| | Acyl | –R | m.p. °C. |
|---|---|---|---|
| (h) | 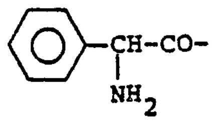 | $-NH_2$ | 216° d. $CF_3COOH$ |
| (i) | 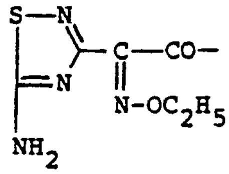 | $-NH_2$ | 204–207° d. |
| (j) | 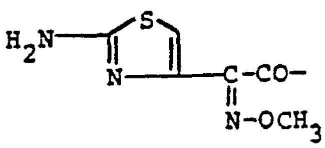 | $-NHCH_2CH_2OCOCH_3$ | 200° d. $K^+$ salt |
| (k) | 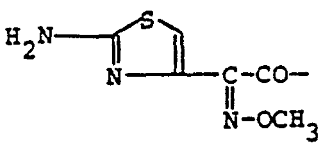 | $-NH-(CH_2)_3-N(CH_3)_2$ | 160° d. |
| (l) | 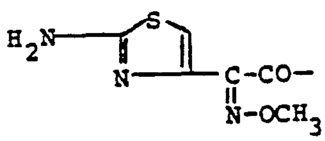 | $-NH-CH_2-CO-NH_2$ | 211–213° $K^+$ salt |
| (m) | $C_6H_5CH_2O-CO-$ | $-NH-NH-COO-tC_4H_9$ | 130° d. |
| (n) | 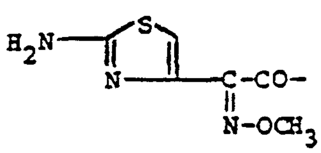 | $-NH-NHCOCH_3$ | 182–200° d. $K^+$ salt |
| (o) | 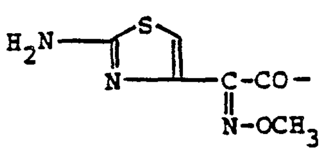 | $-NH-NH_2$ | 150° d. $K^+$ salt |

TABLE 1 (Continued)

| | Acyl | —R | m.p. °C. |
|---|---|---|---|
| (p) | 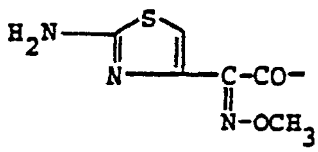 | $-NHCH_2CH_2-$ 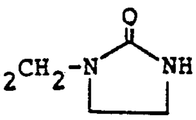 | 179–184 d. $K^+$ salt |
| (q) | 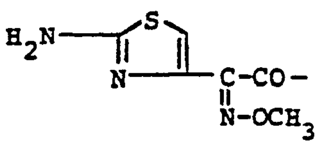 | $-NHCH_2CH_2NHCOCH_3$ | 152 d. $K^+$ salt |

Example 51

By substituting the carboxylic acids containing the acyl groups shown in the first column of Table II into Example 23 for the acid used therein, and substituting the azetidinecarboxamide containing the R-radical indicated for the azetidinecarboxamide used in Example 23, the products shown in Table II are obtained. In the general formula Y can be hydrogen, alkyl, substituted alkyl, phenyl, substituted phenyl, phenylalkyl or (substituted phenyl) alkyl.

TABLE II

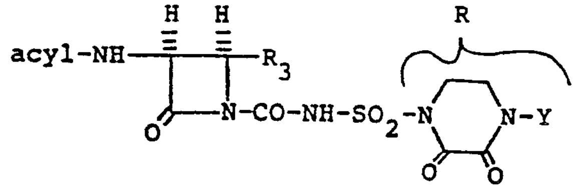

| | Acyl | $R_3$ | Y | m.p. °C. |
|---|---|---|---|---|
| (a) | 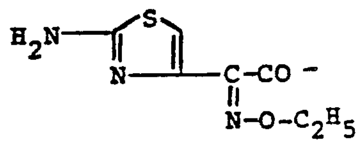 | H | $-C_2H_5$ | 208° d. $K^+$ salt |
| (b) | 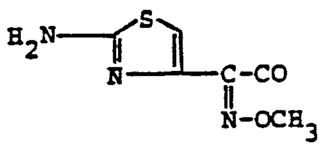 | H | $-C_2H_5$ | 202° d. $K^+$ salt |
| (c) | 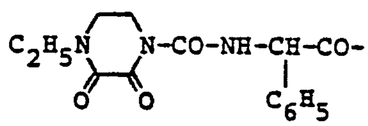 | H | $-C_2H_5$ | 178° d. $K^+$ salt |
| (d) | 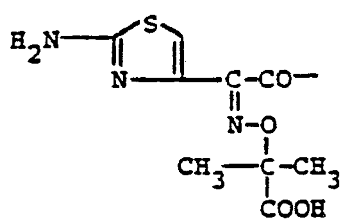 | H | $-C_2H_5$ | 220° d. $K^+$ salt |
| (e) | 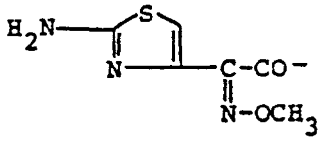 | H | $-iC_3H_7$ | 235–238° $K^+$ salt |
| (f) | 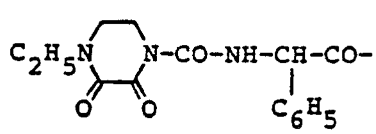 | H | $-iC_3H_7$ | 193° d. $K^+$ salt |

TABLE II (Continued)

| | Acyl | $R_3$ | Y | m.p. °C. |
|---|---|---|---|---|
| (g) | 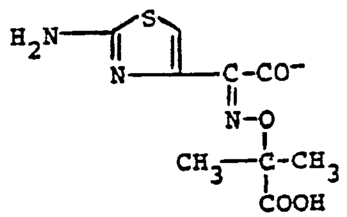 | H | $-iC_3H_7$ | 237° d. $K^+$ salt |
| (h) | 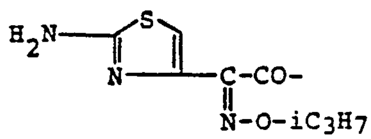 | H | $-C_2H_5$ | 201° d. $K^+$ salt |
| (i) | 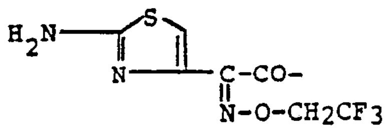 | H | $-C_2H_5$ | 180° d. $K^+$ salt |
| (j) | 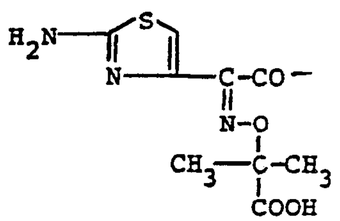 | $-CH_3$ | $-C_2H_5$ | 231° d. $K^+$ salt |
| (k) | 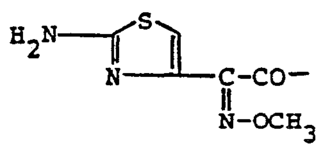 | H | $-C_6H_5$ | 175–180° $K^+$ salt |
| (l) | 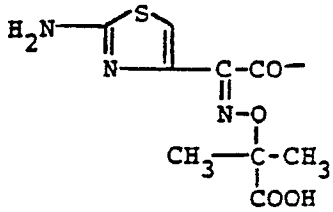 | H | $-C_6H_5$ | 222–228° $K^+$ salt |
| (m) | 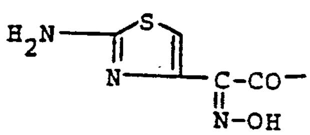 | H | $-C_2H_5$ | 207° d. $K^+$ salt |

Example 52

By substituting the carboxylic acids containing the acyl groups shown in the first column of Table III into Example 23 for the acid used therein, and substituting the azetidinecarboxamide containing the R-radical indicated for the azetidinecarboxamide used in Example 23, the products shown in Table III are obtained. In the general formula, n can be zero or one and X can be methylene, oxygen or a nitrogen atom which can be substituted with alkyl, substituted alkyl, phenyl, substituted phenyl, phenylalkyl, (substituted phenyl)alkyl, amino, alkylamino, dialkylamino, alkylidenamino, aralkylidenamino, alkylsulfonyl, carboxy, carboxyalkyl and the like.

TABLE III

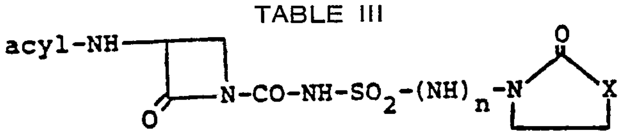

| | Acyl | X | n | m.p. °C. |
|---|---|---|---|---|
| (a) | $C_6H_5CH_2OCO-$ | N–H | 0 | 158 d. $K^+$ salt |
| (b) | 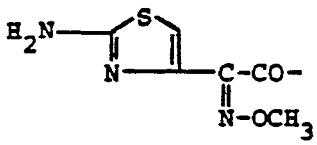 | N–H | 0 | 252 d. $K^+$ salt |
| (c) | 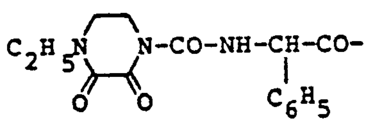 | N–H | 0 | 187 d. $K^+$ salt |
| (d) | 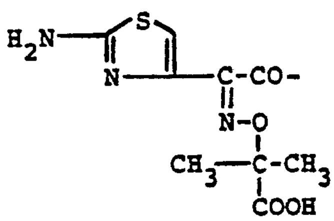 | N–H | 0 | 230 |
| (e) | 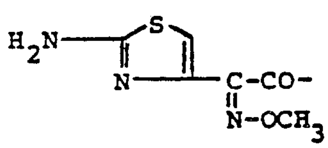 | $N-SO_2CH_3$ | 0 | 210 d. $K^+$ salt |
| (f) | 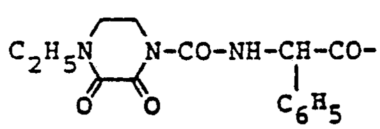 | $N-SO_2CH_3$ | 0 | 187 d. $K^+$ salt |
| (g) | 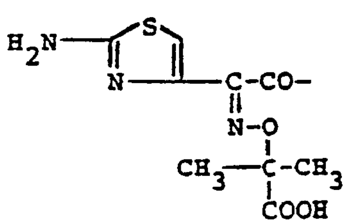 | $N-SO_2CH_3$ | 0 | 227 $K^+$ salt |

TABLE III (Continued)

| | Acyl | X | n | m.p. °C. |
|---|---|---|---|---|
| (h) | 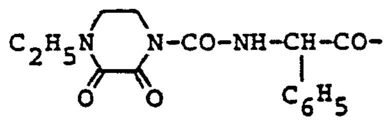 | n–i$C_3H_7$ | 0 | 183 d. $K^+$ salt |
| (i) | 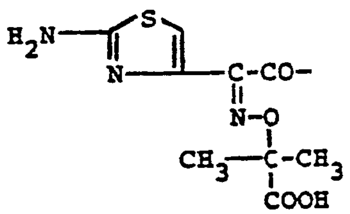 | N–i$C_3H_7$ | 0 | 252 d. $K^+$ salt |
| (j) | 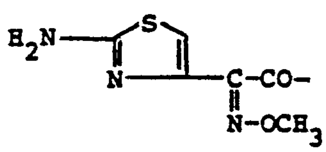 | N–N=CH–$C_6H_5$ | 0 | 253 d. $K^+$ salt |
| (k) | 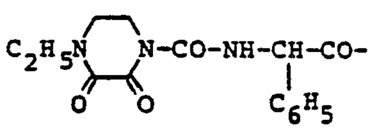 | N–N=CH–$C_6H_5$ | 0 | 176 d. $K^+$ salt |
| (l) | 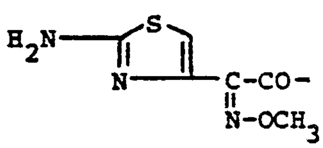 | N–$C_2H_5$ | 0 | 183 d. $K^+$ salt |
| (m) | 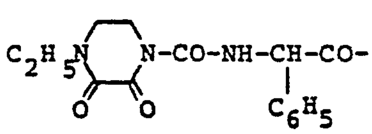 | N–$C_2H_5$ | 0 | 179 d. $K^+$ salt |
| (n) | 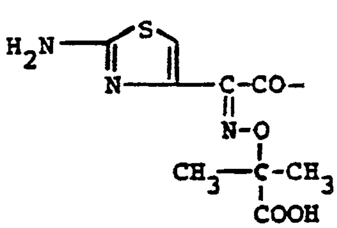 | N–$C_2H_5$ | 0 | 232 d. $K^+$ salt |

TABLE III (Continued)

| | Acyl | X | n | m.p. °C. |
|---|---|---|---|---|
| (o) | 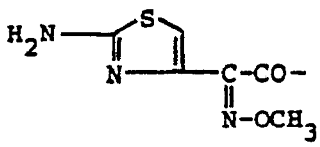 | $N-C_6H_5$ | 0 | 222 d. $K^+$ salt |
| (p) | 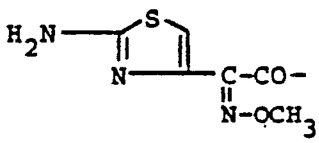 | $N-CH_2-C_6-H_5$ | 0 | 197 d. $K^+$ salt |
| (q) | 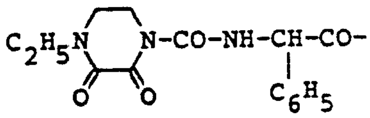 | $N-C_6H_5$ | 0 | 186 d. $K^+$ salt |
| (r) | 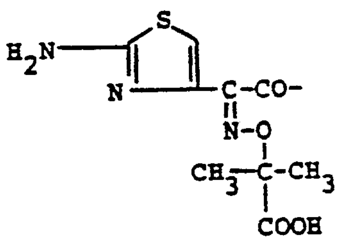 | $N-C_6H_5$ | 0 | 230 d. $K^+$ salt |
| (s) | 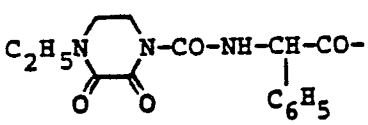 | $N-CH_2C_6H_5$ | 0 | 175 d. $K^+$ salt |
| (t) | 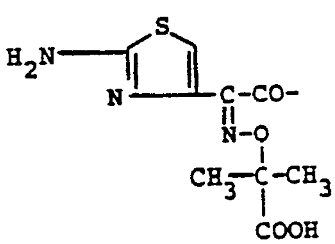 | $N-CH_2-C_6H_5$ | 0 | 207 d. $K^+$ salt |
| (u) | 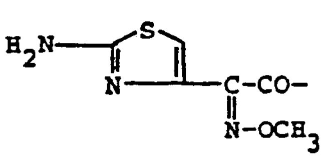 | $N-COOtC_4H_9$ | 0 | 170–175 d. $K^+$ salt |

TABLE III (Continued)

| | Acyl | X | n | m.p. °C. |
|---|---|---|---|---|
| (v) | 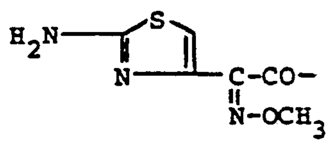 | $N-CH_2CH_2NH_2$ | 0 | 198–203 d. $K^+$ salt |
| (w) | 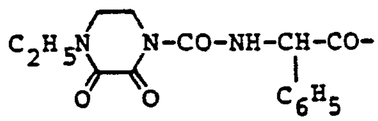 | $N-CH_2CH_2NH_2$ | 0 | 188–193 d. $K^+$ salt |
| (x) | 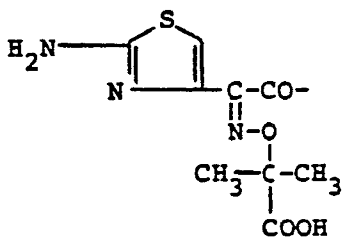 | $N-CH_2CH_2NH_2$ | 0 | 202–205 d. $K^+$ salt |
| (y) | 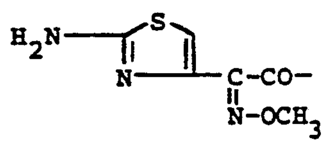 | $N-NH_2$ | 0 | 190–195 d. $K^+$ salt |
| (z) | 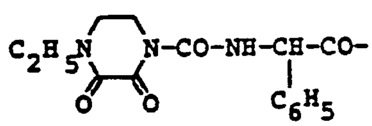 | $N-N=iC_3H_7$ | 0 | 183–188 d. $K^+$ salt |
| (aa) | 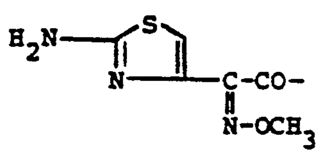 | $CH_2$ | 0 | 200 d. $K^+$ salt |
| (bb) | 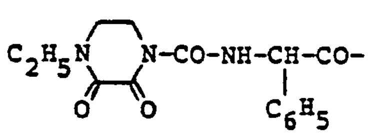 | $CH_2$ | 0 | 187 d. $K^+$ salt |

TABLE III (Continued)

| | Acyl | X | n | m.p. °C. |
|---|---|---|---|---|
| (cc) | 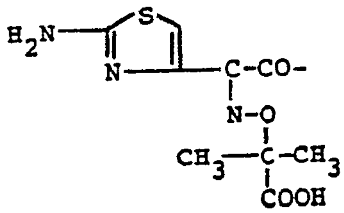 | $CH_2$ | 0 | 245 d. $K^+$ salt |
| (dd) | 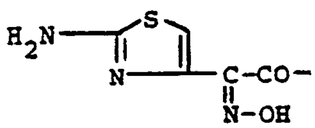 | $CH_2$ | 0 | 202 $K^+$ salt |
| (ee) | 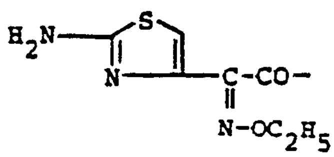 | $CH_2$ | 0 | 195 d. $K^+$ salt |
| (ff) | 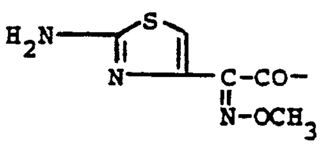 | O | 0 | 184 d. $K^+$ salt |
| (gg) | 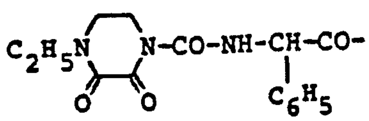 | O | 0 | 176 d. $K^+$ salt |
| (hh) | 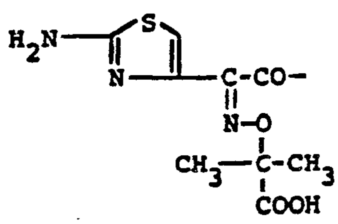 | O | 0 | 259 d. $K^+$ salt |
| (ii) | 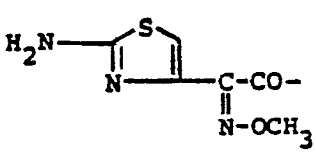 | O | 1 | 200 d. $K^+$ salt |
| (jj) | 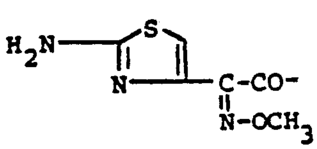 | NH | 1 | 220 d. $K^+$ salt |

Example 53

By substituting the carboxylic acids containing the acyl groups shown in the first column of Table IV into Example 23 for the acid used therein, and substituting the azetidinecarboxamide containing the R-radical indicated for the azetidine carboxamide used in Example 23, the products shown in Table IV are obtained. In the general formula, W can be hydrogen, alkyl, substituted alkyl, phenyl, substituted phenyl, phenylalkyl or (substituted phenyl)alkyl.

TABLE IV

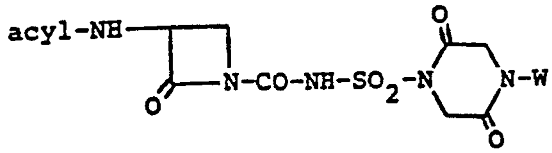

| | Acyl | W | m.p. °C. |
|---|---|---|---|
| (a) | 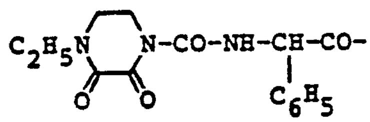 | H | 221 d. $K^+$ salt |
| (b) | 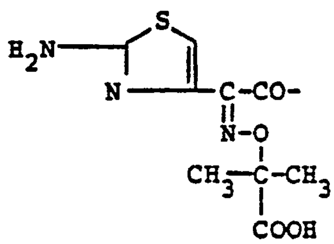 | H | 198 d. $K^+$ salt |
| (c) | 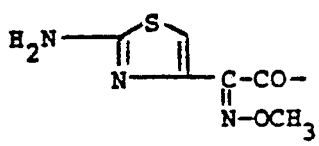 | H | 247 d. $K^+$ salt |
| (d) | 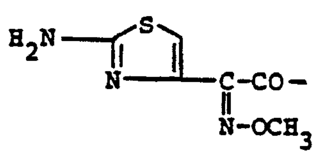 | $CH_3$ | 212 d. $K^+$ salt |
| (e) | 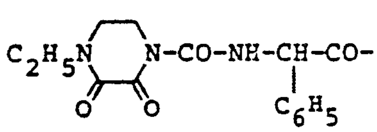 | $CH_3$ | 197 d. $K^+$ salt |
| (f) | 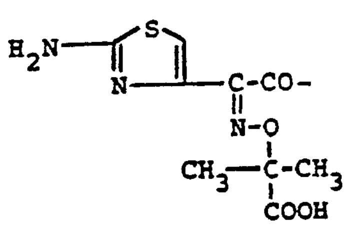 | $CH_3$ | 235 d. $K^+$ salt |

**Claims**

1. A β-lactam having the formula (I)

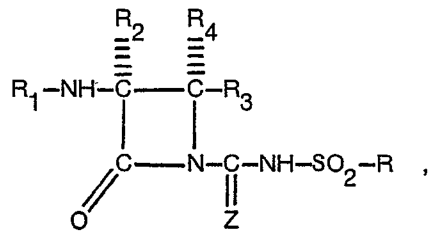

I

or a salt thereof; wherein

R is alkyl, alkenyl, alkynyl, substituted alkyl, phenyl, substituted phenyl, het which is a 5, 6 or 7-membered aromatic, fully or partially saturated heterocycle containing one or more nitrogen, oxygen or sulfur atoms optionally substituted with one or more halogen, hydroxyl, nitro, amino, cyano, trifluoromethyl, $C_1$—$C_4$-alkyl, $C_1$—$C_4$-alkoxy, alkylsulfonyl, phenyl, substituted phenyl, 2-furylimino, benzylimino or substituted $C_1$—$C_4$-alkyl groups, phenylalkyl, (substituted-phenyl)alkyl, het-alkyl or —$NR_aR_b$ wherein $R_a$ and $R_b$ are the same or different and each is hydrogen, alkyl, substituted alkyl, phenyl, substituted phenyl, phenylalkyl, (substituted phenyl)alkyl or —$NR_aR_b$ in which one of $R_a$ and $R_b$ is hydrogen, alkyl, phenyl, substituted phenyl, phenylalkyl or (substituted phenyl)alkyl and the other is amino, alkanoylamino, arylcarbonylamino, alkoxycarbonylamino, alkylsulfonylamino, alkylamino, dialkylamino, phenylamino, (substituted phenyl)amino, hydroxy, cyano, alkoxy, phenyloxy, (substituted phenyl)oxy, phenylalkoxy, (substituted phenyl)alkoxy, het as defined above, het-alkyl, het-alkoxy, alkylsulfonyl, alkylmethyleneamino, phenylmethyleneamino or (substituted phenyl)methyleneamino;

$R_1$ is acyl derived from a carboxylic acid;

$R_2$ is hydrogen or methoxy;

$R_3$ and $R_4$ are the same or different and each is hydrogen, alkyl, cycloalkyl, phenyl or substituted phenyl, or one of $R_3$ and $R_4$ is hydrogen and the other is alkoxycarbonyl, alken-1-yl, alkyn-1-yl, 2-phenylethenyl, 2-phenylethynyl, carboxyl, —$CH_2X_1$ (wherein $X_1$) is azido, amino, hydroxy, alkanoylamino, alkylsulfonyloxy, phenylsulfonyloxy, (substituted phenyl)sulfonyloxy, phenyl, substituted phenyl, halogen, benzylthio (substituted phenyl)methylthio, triphenylmethylthio, cyano or mercapto), —S—$X_2$ or —O—$X_2$ (wherein $X_2$ is alkyl, phenyl, substituted phenyl, alkanoyl, phenylcarbonyl, (substituted phenyl)carbonyl, or heteroarylcarbonyl), or

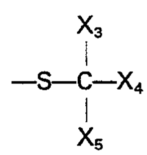

or

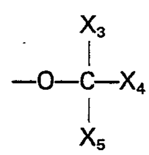

(wherein one of $X_3$ and $X_4$ is hydrogen and the other is hydrogen or alkyl, or $X_3$ and $X_4$ when taken together with the carbon atom to which they are attached form a cycloalkyl group, and $X_5$ is formyl, alkanoyl, phenylcarbonyl, (substituted phenyl)carbonyl, phenylalkylcarbonyl, (substituted phenyl)alkylcarbonyl, carboxyl, alkoxycarbonyl, aminocarbonyl, (substituted amino)carbonyl, or cyano); and

Z is oxygen or sulfur, in which the above definitions are precised as follows:

alkyl and alkoxy groups contain 1 to 10 carbon atoms, cycloalkyl groups contain 3 to 7 carbon atoms, alkanoyl, alkenyl, alkynyl, alken-1-yl and alkyn-1-yl groups contain 2 to 10 carbon atoms,

substituted phenyl refers to a phenyl group substituted with 1, 2 or 3 amino, halogen, hydroxyl, trifluoromethyl, $C_1$—$C_4$ alkyl, $C_1$—$C_4$ alkoxy or carboxyl,

substituted alkyl refers to $C_1$—$C_{10}$ alkyl groups substituted with one, or more, azido, amino, alkylamino, dialkylamino, aralkylamino, halogen, hydroxy, carboxy, cyano, alkoxycarbonyl, alkanoyloxy, alkoxy, phenyloxy, aminocarbonyl, (substituted phenyl)oxy, (heteroaryl)oxy, mercapto, alkylthio, phenylthio, (substituted phenyl)thio, alkylsulfinyl or alkylsulfonyl,

substituted amino refers to a group having the formula —$NY_1Y_2$ wherein $Y_1$ is hydrogen, alkyl, phenyl, substituted phenyl, phenylalkyl or (substituted phenyl)alkyl, and $Y_2$ is alkyl, phenyl, substituted phenyl, phenylalkyl, (substituted phenyl)alkyl, hydroxy, cyano, alkoxy, phenylalkoxy, or amino (—$NH_2$).

2. A β-lactam in accordance with claim 1 wherein $R_2$ is hydrogen.

3. A β-lactam in accordance with claim 2 wherein $R_3$ is hydrogen and $R_4$ is hydrogen or methyl.

4. A β-lactam in accordance with claim 3, wherein Z is oxygen.

5. A β-lactam in accordance with claim 1 wherein Z is oxygen.

6. A β-lactam in accordance with claim 1 having the formula (II)

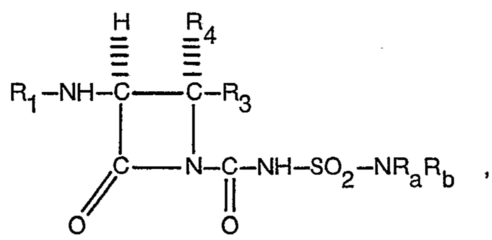

II

or a salt thereof.

7. A β-lactam in accordance with claim 6 wherein $R_a$ and $R_b$ each is hydrogen.

8. A β-lactam in accordance with claim 1 wherein $R_1$ is

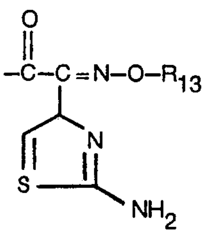

and $R_{13}$ is alkyl or carboxy substituted alkyl.

9. A β-lactam having the formula (III)

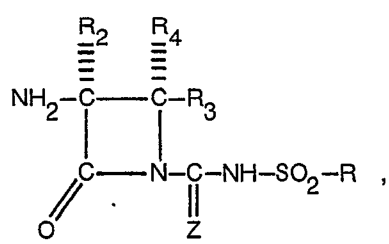

III

or a salt thereof; wherein R, $R_2$, $R_3$, $R_4$ and Z are as defined in claim 1.

10. A β-lactam in accordance with claim 9 wherein $R_2$ is hydrogen.

11. The compound in accordance with 1, (S)-[1-[[[(4-methylphenyl)sulfonyl]amino]carbonyl]-2-oxo-3-azetidinyl]carbamic acid, phenylmethyl ester.

12. The compound in accordance with claim 1, [3S(Z)]-3-[[(2-amino-4-thiazolyl)(methoxyimino)acetyl]-amino]-N-[(4-methylphenyl)sulfonyl]-2-oxo-1-azetidinecarboxamide.

13. The compound in accordance with claim 1, (3S(Z)]-3-[[(2-amino-4-thiazolyl)(methoxyimino)-acetyl]amino]-N-(methylsulfonyl)-2-oxo-1-azetidinecarboxamide, potassium salt.

14. The compound in accordance with claim 1, [3S-[3α(Z),4β]]-3-[[(2-amino-4-thiazolyl)(methoxy-imino)acetyl]amino]-4-methyl-N-(methylsulfonyl)-2-oxo-1-azetidinecarboxamide, potassium salt.

15. The compound in accordance with claim 1, [3S-[3α(Z),4β]]-3-[[(2-amino-4-thiazolyl)[1-carboxy-1-methylethoxy)imino]acetyl]amino]-4-methyl-N-(methylsulfonyl)-2-oxo-1-azetidinecarboxamide, dipotassium salt.

16. The compound in accordance with claim 1, [3S(Z)]-3-[[(2-amino-4-thiazolyl)(methoxyimino)acetyl]-amino]-N-(methylsulfonyl)-2-oxo-1-azetidinecarbothioamide, potassium salt.

17. The compound in accordance with claim 1, [3S(Z)]-2-[[[1-(2-amino-4-thiazolyl)-2-[[1-[[(methylsulfonyl)amino]carbonyl]-2-oxo-3-azetidinyl]amino]-2-oxoethylidene]amino]oxy]-2-methylpropanoic acid, dipotassium salt.

18. The compound in accordance with claim 1, [3S(Z)]-3-[[(2-amino-4-thiazolyl)(methoxyimino)acetyl]-amino]-N-(aminosulfonyl)-2-oxo-1-azetidinecarboxamide, ammonium salt.

19. The compound in accordance with claim 1, [3S(Z)]-3-[[(2-amino-4-thiazolyl)(methoxyimino)acetyl]-amino]-N-[[(1-methylethyl)amino]sulfonyl]-2-oxo-1-azetidinecarboxamide, sodium salt.

20. The compound in accordance with claim 1, [3S(Z)]-3-[[(2-amino-4-thiazolyl)(methoxyimino)acetyl]-amino]-2-oxo-N-[(phenylamino)sulfonyl]-1-azetidinecarboxamide, sodium salt.

21. The compound in accordance with claim 1, [3S(Z)]-3-[[(2-amino-4-thiazolyl)(methoxyimino)acetyl]-amino]-N-[(dimethylamino)sulfonyl]-2-oxo-1-azetidinecarboxamide, sodium salt.

22. The compound in accordance with claim 1, [3S(Z)]-2-[[[1-(2-amino-4-thiazolyl)-2-[[1-[[(aminosulfonyl)amino]carbonyl]-2-oxo-3-azetidinyl]amino]-2-oxoethylidene]amino]oxy]-2-methylpropanoic acid, disodium salt.

23. The compound in accordance with claim 9, (S)-N-(methylsulfonyl)-2-oxo-3-amino-1-azetidinecarboxamide, hydrochloride salt.

24. The compound in accordance with claim 9, (S)-3-amino-N-(methylsulfonyl)-2-oxo-1-azetidinecarboxamide, inner salt.

25. The compound in accordance with claim 9, [3S-[3α,4β]]-3-amino-4-methyl-N-(methylsulfonyl)-2-oxo-1-azetidinecarboxamide, trifluoroacetic acid salt.

26. The compound in accordance with claim 9, (S)-3-amino-N-(methylsulfonyl)-2-oxo-1-azetidinecarbothioamide, trifluoroacetic acid salt.

27. The compound in accordance with claim 9, (S)-3-amino-N-(aminosulfonyl)-2-oxo-1-azetidinecarboxamide, inner salt.

28. The compound in accordance with claim 9, (S)-3-amino-N-[(methylamino)sulfonyl]-2-oxo-1-azetidinecarboxamide, inner salt.

29. The compound in accordance with claim 9, (S)-3-amino-2-oxo-N-[[(4-pyridyl)amino]sulfonyl]-1-azetidinecarboxamide.

30. The compound in accordance with claim 9, (S)-1-[[[(4-methylphenyl)sulfonyl]amino]carbonyl]-2-oxo-3-aminoazetidine, triethylammonium salt.

31. A compound according to claim 1 of the formula (IV)

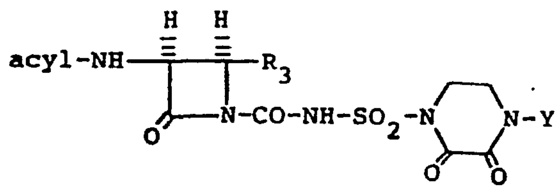

(IV)

wherein Y can be hydrogen, alkyl, substituted alkyl, phenyl, substituted phenyl, phenylalkyl or (substituted phenyl)alkyl in which the above definitions are precised as in claim 1.

32. A compound according to claim 1 of the formula (V)

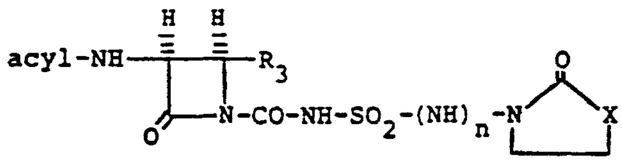

(V)

wherein n can be zero or one and X can be methylene, oxygen or a nitrogen atom which can be substituted with alkyl, substituted alkyl, phenyl, substituted phenyl, phenylalkyl, (substituted phenyl)-alkyl, amino, alkylamino, dialkylamino, alkylidenamino, aralkylidenamino, alkylsulfonyl, carboxy, carboxyalkyl in which the above definitions are precised as in claim 1.

33. A compound according to claim 1 of the formula (VI)

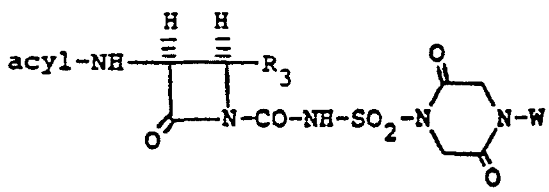

(VI)

wherein W can be hydrogen, alkyl, substituted alkyl, phenyl, substituted phenyl, phenylalkyl or (substituted phenyl)alkyl in which the above definitions are precised as in claim 1.

**Patentansprüche**

1. β-Lactam mit der allgemeinen Formel I

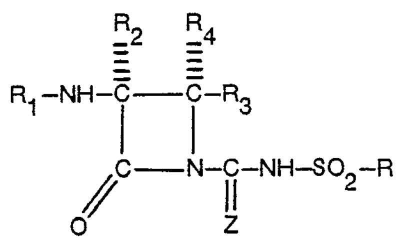

I

oder ein Salz davon, in der

R einen Alkylrest, Alkenylrest, Alkinylrest, einen substituierten Alkylrest, eine Phenylgruppe, einen substituierten Phenylrest, einen Rest het, der einen heterocyclischen, ganz oder teilweise gesättigten aromatischen 5-, 6- oder 7-Ring mit mindestens einem Stickstoff-, Sauerstoff- oder Schwefelatom, gegebenenfalls substituiert mit einem oder mehreren Halogenatomen, Hydroxylgruppen, Nitrogruppen, Aminogruppen, Cyangruppen, Trifluormethylgruppen, $C_1$—$C_4$-Alkylresten, $C_1$—$C_4$-Alkoxyresten, Alkylsulfonylresten, Phenylgruppen, substituierten Phenylresten, 2-Furyliminogruppen, Benzyliminogruppen oder substituierten $C_1$—$C_4$-Alkylresten darstellt, einen Phenylalkylrest, einen substituierten Phenylalkylrest, einen Rest het-Alkyl oder einen Rest —$NR_aR_b$, in dem $R_a$ und $R_b$ gleich oder verschieden sind und jeweils ein Wasserstoffatom, einen Alkylrest, einen substituierten Alkylrest, eine Phenylgruppe, einen substituierten Phenylrest, einen Phenylalkylrest oder einen substituierten Phenylalkylrest darstellen, oder einen Rest —$NR_aR_b$ bedeutet, in dem einer der Reste $R_a$ oder $R_b$ ein Wasserstoffatom, einen Alkylrest, eine Phenylgruppe, einen substituierten Phenylrest, einen Phenylalkylrest oder einen substituierten Phenylalkylrest bedeutet und der andere Rest eine Aminogruppe, einen Alkanoylaminorest, einen Arylcarbonylaminorest, einen Alkoxycarbonylaminorest, einen Alkylsulfonylaminorest, einen Alkylaminorest, einen Dialkylaminorest, eine Phenylaminogruppe, einen substituierten Phenylaminorest, eine Hydroxylgruppe, eine Cyangruppe, einen Alkoxyrest, eine Phenyloxygruppe, einen substituierten Phenyloxyrest, einen Phenylalkoxyrest, einen substituierten Phenylalkoxyrest, einen wie vorstehend definierten Rest het, einen Rest het-Alkyl, einen Rest het-Alkoxy, einen Alkylsulfonylrest, einen Alkylmethylenaminorest, eine Phenylmethylenaminogruppe oder einen substituierten Phenylmethylenaminorest darstellt,

$R_1$ ein von einer Carbonsäure stammender Acylrest ist,

$R_2$ ein Wasserstoffatom oder eine Methoxygruppe darstellt,

$R_3$ und $R_4$ gleich oder verschieden sind und jeweils ein Wasserstoffatom, einen Alkylrest, einen Cycloalkylrest, eine Phenylgruppe oder einen substituierten Phenylrest bedeuten, oder einer der Reste $R_3$ und $R_4$ ein Wasserstoffatom und der andere einen Alkoxycarbonylrest, einen Alken-1-ylrest, einen Alkin-1-ylrest, eine 2-Phenyläthenylgruppe, eine 2-Phenyläthinylgruppe, eine Carboxylgruppe, einen Rest

$$—CH_2X_1$$

(in dem $X_1$ eine Azido-, Amino-, Hydroxyl-, Alkanoylamino-, Alkylsulfonyloxy-, Phenylsulfonyloxy-, substituierte Phenylsulfonyloxy-, Phenyl-, substituierte Phenyl-, Halogen-, Benzylthio-, substituiertes Phenylmethylthio-, Triphenylmethylthio-, Cyan- oder Mercaptogruppe bedeutet), einen Rest

$$—S—X_2 \text{ oder } —O—X_2$$

(in dem $X_2$ einen Alkyl-, Phenyl-, substituierten Phenyl-, Alkanoyl-, Phenylcarbonyl-, substituierte Phenylcarbonyl- oder Heteroarylcarbonylrest bedeutet) oder einen Rest

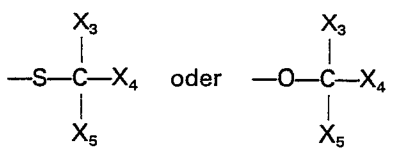

(in denen einer der Rest $X_3$ und $X_4$ ein Wasserstoffatom und der andere ein Wasserstoffatom oder einen Alkylrest bedeutet, oder $X_3$ und $X_4$ zusammen mit dem Kohlenstoffatom, an das sie gebunden sind, einen Cycloalkylrest bilden, und $X_5$ einen Formyl-, Alkanoyl, Phenylcarbonyl-, substituierten Phenylcarbonyl-, Phenylalkylcarbonyl-, substituierten Phenylalkylcarbonyl-, Carboxyl-, Alkoxycarbonyl-, Aminocarbonyl-, substituierten Aminocarbonyl- oder Cyanrest bedeutet), darstellt, und Z ein Sauerstoffatom oder ein Schwefelatom ist, wobei die vorstehenden Definitionen die folgenden Bedeutungen haben:

Alkyl- und Alkoxyrest enthalten 1 bis 10 Kohlenstoffatome, Cycloalkylreste enthalten 3 bis 7 Kohlenstoffatome, Alkanoyl-, Alkenyl-, Alkinyl-, Alken-1-yl- und Alkin-1-ylreste enthalten 2 bis 10 Kohlenstoffatome, substituierter Phenylrest bedeutet einen Phenylrest, der mit 1, 2 oder 3 Aminogruppen, Halogenatomen, Hydroxylgruppen, Trifluormethylgruppen, $C_1$—$C_4$-Alkylresten, $C_1$—$C_4$-Alkoxyresten oder Carboxylgruppen substituiert ist,

substituierte Alkylreste bedeutet $C_1$—$C_{10}$-Alkylreste, die mit einem oder mehreren Azido-, Amino-, Alkylamino-, Dialkylamino-, Aralkylamino-, Halogen-, Hydroxyl-, Carboxyl-, Cyan-, Alkoxycarbonyl-, Alkanoyloxy-, Alkoxy-, Phenyloxy-, Aminocarbonyl-, substituierten Phenyloxy-, Heteroaryloxy-, Mercapto-, Alkylthio-, Phenylthio-, substituierten Phenylthio-, Alkylsulfinyl- oder Alkylsulfonylresten substituiert sind, eine substituierte Aminogruppe bedeutet einen Rest mit der allgemeinen Formel —$NY_1Y_2$, in der $Y_1$ ein Wasserstoffatom, ein Alkylrest, eine Phenylgruppe, ein substituierter Phenylrest, ein Phenylalkylrest oder ein substituierter Phenylalkylrest ist und $Y_2$ einen Alkylrest, Phenylgruppe, substituierten Phenylrest, Phenylalkylrest, substituierten Phenylalkylrest, Hydroxylgruppe, Cyangruppe, Alkoxyrest, Phenylalkoxyrest oder eine Aminogruppe (—$NH_2$) bedeutet.

2. β-Lactam nach Anspruch 1, in dem $R_2$ ein Wasserstoffatom bedeutet.

3. β-Lactam nach Anspruch 2, in dem $R_3$ ein Wasserstoffatom und $R_4$ ein Wasserstoffatom oder eine Methylgruppe bedeutet.

4. β-Lactam nach Anspruch 3, in dem Z ein Sauerstoffatom bedeutet.

5. β-Lactam nach Anspruch 1, in dem Z ein Sauerstoffatom bedeutet.

6. β-Lactam nach Anspruch 1 mit der allgemeinen Formel II

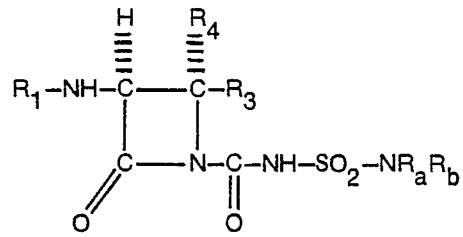

II

oder ein Salz davon.

7. β-Lactam nach Anspruch 6, in dem $R_a$ und $R_b$ jeweils ein Wasserstoffatom bedeuten.

8. β-Lactam nach Anspruch 1, in dem $R_1$ den Rest

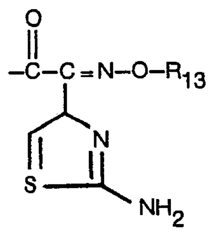

darstellt und $R_{13}$ einen Alkylrest oder einen mit einer Carboxylgruppe substituierten Alkylrest bedeutet.

9. β-Lactam der allgemeinen Formel III

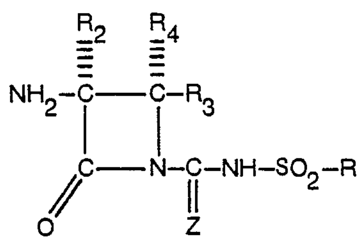

III

oder ein Salz davon, in der R, $R_2$, $R_3$, $R_4$ und Z die in Anspruch 1 angegebene Bedeutung haben.

10. β-Lactam nach Anspruch 9, in dem $R_2$ ein Wasserstoffatom bedeutet.

11. Verbindung nach Anspruch 1, nämlich der Phenylmethylester der (S)-[1-[[[(4-Methylphenyl)-sulfonyl]-amino]-carbonyl]-2-oxo-3-azetidinyl]carbaminsäure.

12. Verbindung nach Anspruch 1, nämlich [3S(Z)]-3-[[(2-Amino-4-thiazolyl)-(methoxyimino)-acetyl]-amino]-N-[(4-methylphenyl)-sulfonyl]-2-oxo-1-azetidinecarboxamid.

13. Verbindung nach Anspruch 1, nämlich das Kaliumsalz von (3S(Z)]-3-[[(2-Amino-4-thiazolyl)-methoxyimino)-acetyl]-amino]-N-(methylsulfonyl)-2-oxo-1-azetidincarboxamid.

14. Verbindung nach Anspruch 1, nämlich das Kaliumsalz von [3S-[3α(Z),4β]]-3-[[(2-Amino-4-thiazolyl)-(methoxyimino)-acetyl]-amino]-4-methyl-N-(methylsulfonyl)-2-oxo-1-azetidinecarboxamid.

15. Verbindung nach Anspruch 1, nämlich das Di-Kaliumsalz von [3S-[3α(Z),4β]]-3-[[(2-Amino-4-thiazolyl)-[(1-carboxy-1-methyläthoxy)-imino]-acetyl]-amino]-4-methyl-N-(methylsulfonyl)-2-oxo-1-azetidinecarboxamid.

16. Verbindung nach Anspruch 1, nämlich das Kaliumsalz von [3S(Z)]-3-[[(2-Amino-4-thiazolyl)-(methoxyimino)-acetyl]-amino]-N-(methylsulfonyl)-2-oxo-1-azetidincarbothioamid.

17. Verbindung nach Anspruch 1, nämlich das Di-Kaliumsalz der [3S(Z)]-2-[[[1-(2-Amino-4-thiazolyl)-2-[[1-[[(methylsulfonyl)-amino]-carbonyl]-2-oxo-3-azetidinyl]-amino]-2-oxoäthyliden]-amino]-oxy]-2-methyl-propionsäure.

18. Verbindung nach Anspruch 1, nämlich das Ammoniumsalz von [3S(Z)]-3-[[(2-Amino-4-thiazolyl)-(methoxyimino)-acetyl]-amino]-N-(aminosulfonyl)-2-oxo-1-azetidincarboxamid.

19. Verbindung nach Anspruch 1, nämlich das Natriumsalz von [3S(Z)]-3-[[(2-Amino-4-thiazolyl)-(methoxyimino)-acetyl]-amino]-N-[[(1-methyläthyl)-amino]-sulfonyl]-2-oxo-1-azetidincarboxamid.

20. Verbindung nach Anspruch 1, nämlich das Natriumsalz von [3S(Z)]-3-[[(2-Amino-4-thiazolyl)-(methoxyimino)-acetyl]-amino]-2-oxo-N-[(phenylamino)-sulfonyl)-1-azetidincarboxamid.

21. Verbindung nach Anspruch 1, nämlich das Natriumsalz von [3S(Z)]-3-[[(2-Amino-4-thiazolyl)-(methoxyimino)-acetyl]-amino]-N-[(dimethylamino)-sulfonyl]-2-oxo-1-azetidincarboxamid.

22. Verbindung nach Anspruch 1, nämlich das Di-Natriumsalz der [3S(Z)]-2-[[[1-(2-Amino-4-thiazolyl)-2-[[1-[[(aminosulfonyl)-amino]-carbonyl]-2-oxo-3-azetidinyl]-amino]-2-oxoäthyliden]-amino]-oxy]-2-methyl-propionsäure.

23. Verbindung nach Anspruch 9, nämlich das Hydrochlorid von (S)-N-(Methylsulfonyl)-2-oxo-3-amino-1-azetidincarboxamid.

24. Verbindung nach Anspruch 9, nämlich das Innere Salz von (S)-3-Amino-N-(methylsulfonyl)-2-oxo-1-azetidincarboxamid.

25. Verbindung nach Anspruch 9, nämlich das Trifluoressigsäure-Salz von [3S-[3α,4β]]-3-Amino-4-methyl-N-(methylsulfonyl)-2-oxo-1-azetidincarboxamid.

26. Verbindung nach Anspruch 9, nämlich das Trifluoressigsäure-Salz von (S)-3-Amino-N-(methylsulfonyl)-2-oxo-1-azetidin-carbothioamid.

27. Verbindung nach Anspruch 9, nämlich das innere Salz von (S)-3-Amino-N-(aminosulfonyl)-2-oxo-1-azetidincarboxamid.

28. Verbindung nach Anspruch 9, nämlich das innere Salz von (S)-3-Amino-N-[(methylamino)-sulfonyl]-2-oxo-1-azetidincarboxamid.

29. Verbindung nach Anspruch 9, nämlich (S)-3-Amino-2-oxo-N-[[(4-pyridyl)-amino]-sulfonyl]-1-azetidincarboxamid.

30. Verbindung nach Anspruch 9, nämlich das Triäthylammonium-Salz von (S)-1-[[[(4-Methylphenyl)-sulfonyl]-amino]-carbonyl]-2-oxo-3-aminoazetidin.

31. Verbindung nach Anspruch 1 mit der allgemeinen Formel IV

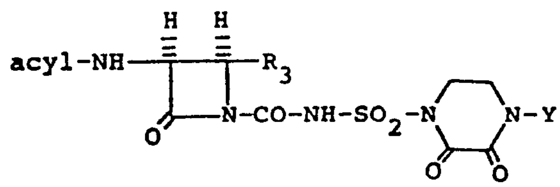

(IV)

in der Y ein Wasserstoffatom, ein Alkylrest, ein substituierter Alkylrest, eine Phenylgruppe, ein substituierter Phenylrest, ein Phenylalkylrest oder ein substituierter Phenylalkylrest sein kann, wobei die vorstehenden Definitionen die in Anspruch 1 angegebene genaue Bedeutung haben.

32. Verbindung nach Anspruch 1 mit der allgemeinen Formel V

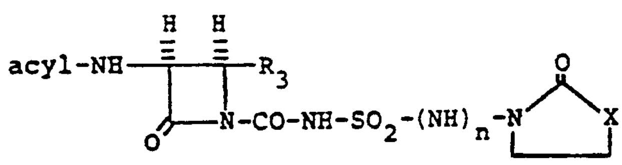

(V)

in der n den Wert 0 oder 1 haben kann und X eine Methylengruppe, ein Sauerstoffatom oder ein Stickstoffatom sein kann, welches mit einem Alkylrest, einem substituierten Alkylrest, einer Phenylgruppe, einem substituierten Phenylrest, einem Phenylalkylrest, einem substituierten Phenylalkylrest, einer Aminogruppe, einem Alkylaminorest, einem Dialkylaminorest, einem Alkylidenaminorest einem Aralkylidenaminorest, einem Alkylsulfonylrest, einer Carboxylgruppe oder einem Carboxyalkylrest substituiert sein kann, wobei die vorstehenden Definitionen die in Anspruch 1 angegebene genaue Bedeutung haben.

33. Verbindung nach Anspruch 1 mit der allgemeinen Formel VI

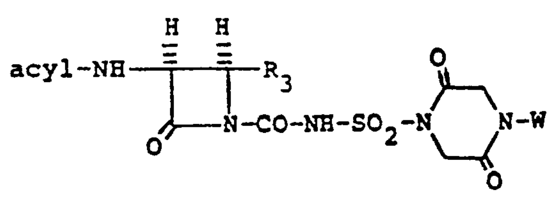

(VI)

in der W ein Wasserstoffatom, ein Alkylrest, ein substituierter Alkylrest, eine Phenylgruppe, ein substituierter Phenylrest, ein Phenylalkylrest oder ein substituierter Phenylalkylrest sein kann, wobei die vorstehenden Definitionen die im Anspruch 1 angegebene genaue Bedeutung haben.

**Revendications**

1. β-lactame de formule (I)

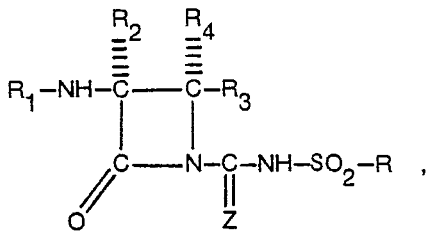

I

éventuellement sous la forme d'un sel, formule dans laquelle

R est un radical alkyle, alcényle, alcynyle, alkyle substitué, phényle, phényle substitué,

het qui est un hétérocycle aromatique, totalement ou partiellement saturé, à 5, 6 ou 7 chaînons, contenant un ou plusieurs atomes d'azote, d'oxygène ou de soufre, éventuellement substitué par un ou plusieurs atomes ou groupements halogène, hydroxyle, nitro, amine, cyano, trifluorométhyle, alkyle en $C_1$—$C_4$, alcoxy en $C_1$—$C_4$, alkylsulfonyle, phényle, phényle substitué, 2-furylimine, benzylimine ou alkyle substitué en $C_1$—$C_{10}$, un radical phénylalkyle, (phényl substitué)alkyle, het-alkyle,

ou —$NR_aR_b$ où $R_a$ et $R_b$ sont identiques ou différents et sont chacun un atome d'hydrogène, ou un radical alkyle éventuellement substitué, phényle éventuellement substitué, phénylalkyle, (phényl substitué) alkyle,

ou un groupement de formule —$NR_aR_b$ dans lequel l'un de $R_a$ et $R_b$ est un atome d'hydrogène ou un radical alkyle, phényle éventuellement substitué, phénylalkyle ou (phényl substitué)alkyle et l'autre est un groupement amine, alcanoylamine, arylcarbonylamine, alcoxycarbonylamine, alkylsulfonylamine, alkylamine, dialkylamine, phénylamine, (phényl substitué)amine, hydroxy, cyano, alcoxy, phényloxy, (phényl substitué)oxy, phénylalcoxy, (phényl substitué)alcoxy, het tel que défini ci-dessus, het-alkyle, het-alcoxy, alkylsulfonyle, alkylméthylèneamine, phénylméthylèneamine ou (phényl substitué)-méthylèneamine;

$R_1$ est un radical acyle dérivé d'un acide carboxylique;

$R_2$ est un atome d'hydrogène ou un radical méthoxy;

$R_3$ et $R_4$ sont identiques ou différents et sont chacun un atome d'hydrogène ou un radical alkyle, cycloalkyle, ou phényle éventuellement substituè,

ou bien l'un de $R_3$ et $R_4$ est un atome d'hydrogène et l'autre est un radical alcoxycarbonyle, alcène-1-yle, alcyn-1-yle, 2-phényléthényle, 2-phényléthynyle, carboxyle,

—$CH_2X_1$ (où $X_1$ est un groupement azide, amine, hydroxy, alcanoylamine, alkylsulfonyloxy, phénylsulfonyloxy, (phényl substitué)sulfonyloxy, phényle, phényle substitué, halogène, benzylthio, (phényl substitué)méthylthio, triphénylméthylthio, cyano ou mercapto)

—S—$X_2$ ou —O—$X_2$ (où $X_2$ est un radical alkyle, phényle éventuellement substitué, alcanoyle, phénylcarbonyle, (phényl substitué)carbonyle, ou hétéroarylcarbonyle, ou

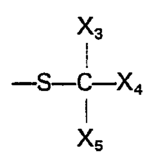 ou 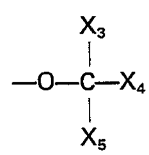

(où l'un de $X_3$ et $X_4$ est un atome d'hydrogène et l'autre est un atome d'hydrogène ou un radical alkyle, ou bien $X_3$ et $X_4$ forment, avec l'atome de carbone auquel ils sont fixés, un radical cycloalkyle, et $X_5$ est un radical formyle, alcanoyle, phénylcarbonyle, (phényl substitué)carbonyle, phénylalkylcarbonyle, (phényl substitué)alkylcarbonyle, carboxyle, alcoxycarbonyle, aminocarbonyle, (amino substitué)carbonyle, ou cyano); et

Z est un atome d'oxygène ou de soufre,

les définitions précédentes étant précisées de la façon suivante:

les radicaux alkyle et alcoxy contiennent de 1 à 10 atomes de carbone, les radicaux cycloalkyle contiennent de 3 à 7 atomes de carbone, les radicaux alcanoyle, alcényle, alcynyle, alcèn-1-yle et alcyn-1-yle contiennent de 2 à 10 atomes de carbones,

les radicaux phényle substitués sont des radicaux phényle substitués par 1, 2 ou 3 atomes ou groupements amine, halogène, hydroxyle, trifluorométhyle, alkyle en $C_1$—$C_4$, alcoxy en $C_1$—$C_4$ ou carboxyle,

les radicaux alkyle substitués sont des radicaux alkyle en $C_1$—$C_{10}$ substitués par un ou plusieurs atomes ou groupements azide, amine, alkylamine, dialkylamine, aralkylamine, halogène, hydroxy, carboxy, cyano, alcoxycarbonyle, alcanoyloxy, alcoxy, phényloxy, aminocarbonyle, (phényl substitué)oxy, hétéroaryloxy, mercapto, alkylthio, phénylthio, (phényl substitué)thio, alkylsulfinyle ou alkylsulfonyle,

les radicaux amine substitués sont des radicaux de formule $-NY_1Y_2$ dans laquelle $Y_1$ est un atome d'hydrogène ou un radical alkyle, phényle éventuellement substitué, phénylalkyle ou (phényl substitué)alkyle, et $Y_2$ est un radical alkyle, phényle éventuellement substitué, phénylalkyle, (phényl substitué)alkyle, hydroxy, cyano, alcoxy, phénylalcoxy, ou amine ($-NH_2$).

2. β-lactame selon la revendication 1, dans la formule duquel $R_2$ est un atome d'hydrogène.

3. β-lactame selon la revendication 2, dans la formule duquel $R_3$ est un atome d'hydrogène et $R_4$ est un atome d'hydrogène ou un radical méthyle.

4. β-lactame selon la revendication 3, dans la formule duquel Z est un atome d'oxygène.

5. β-lactame selon la revendication 1, dans la formule duquel Z est un atome d'oxygène.

6. β-lactame selon la revendication 1, ayant pour formule (II)

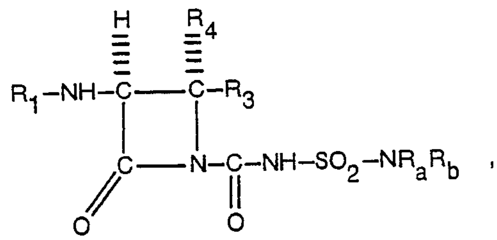

II

éventuellement sous la forme d'un sel.

7. β-lactame selon la revendication 6, dans la formule duquel $R_a$ et $R_b$ sont chacun un atome d'hydrogène.

8. β-lactame selon la revendication 1, dans la formule duquel $R_1$ est un groupement de formule

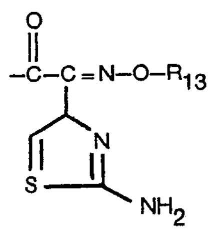

et $R_{13}$ est un radical alkyle éventuellement substitué par un groupement carboxy.

9. β-lactame de formule (III)

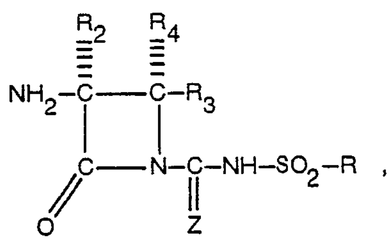

III

éventuellement sous la forme d'un sel, formule dans laquelle R, $R_2$, $R_3$, $R_4$ et Z sont tels que définis dans la revendication 1.

10. β-lactame selon la revendication 9, dans la formule duquel $R_2$ est un atome d'hydrogène.

11. Composé selon la revendication 1, qui est l'ester phénylméthylique de l'acide (S)-[1-[[[(4-méthylphényl)sulfonyl]amino]carbonyl]-2-oxo-3-azétidinyl]carbamique.

12. Composé selon la revendication 1, qui est le [3S(Z)]-3-[[(2-amino-4-thiazolyl)(méthoxyimino)-acétyl]amino]-N-[(4-méthylphényl)sulfonyl]-2-oxo-1-azétidinecarboxamide.

13. Composé selon la revendication 1, qui est le sel de potassium du (3S(Z)]-3-[[(2-amino-4-thiazolyl)-(méthoxyimino)acétyl]amino]-N-(méthylsulfonyl)-2-oxo-1-azétidinecarboxamide.

14. Composé selon la revendication 1, qui est le sel de potassium du [3S-[3α(Z),4β]]-3-[[(2-amino-4-thiazolyl)(méthoxyimino)acétyl]amino]-4-méthyl-N-(méthylsulfonyl)-2-oxo-1-azétidinecarboxamide.

15. Composé selon la revendication 1, qui est le sel dipotassique du [3S-[3α(Z),4β]]-3-[[(2-amino-4-thiazolyl)[(1-carboxy-1-méthyléthoxy)imino]acétyl]amino]-4-méthyl-N-(méthylsulfonyl)-2-oxo-1-azétidine-carboxamide.

16. Composé selon la revendication 1, qui est le sel de potassium du [3S(Z)]-3-[[(2-amino-4-thiazolyl)-(méthoxyimino)acétyl]amino]-N-(méthylsulfonyl)-2-oxo-1-azétidinecarbothioamide.

17. Composé selon la revendication 1, qui est le sel dipotassique de l'acide [3S(Z)]-2-[[[1-(2-amino-4-thiazolyl)-2-[[1-[[(méthylsulfonyl)amino]carbonyl]-2-oxo-3-azétidinyl]amino]-2-oxo-éthylidène]amino]oxy]-2-méthylpropanoïque.

18. Composé selon la revendication 1, qui est le sel d'ammonium du [3S(Z)]-3-[[(2-amino-4-thiazolyl)-(méthoxyimino)acétyl]amino]-N-(aminosulfonyl)-2-oxo-1-azétidinecarboxamide.

19. Composé selon la revendication 1, qui est le sel de sodium du [3S(Z)]-3-[[(2-amino-4-thiazolyl)-(méthoxyimino)acétyl]amino]-N-[[(1-méthyléthyl)amino]sulfonyl]-2-oxo-1-azétidinecarboxamide.

20. Composé selon la revendication 1, qui est le sel de sodium du [3S(Z)]-3-[[(2-amino-4-thiazolyl)-(méthoxyimino)acétyl]amino]-2-oxo-N-[(phénylamino)sulfonyl)-1-azétidinecarboxamide.

21. Composé selon la revendication 1, qui est le sel de sodium du [3S(Z)]-3-[[(2-amino-4-thiazolyl)-(méthoxyimino)acétyl]amino]-N-[(diméthylamino)sulfonyl]-2-oxo-1-azétidinecarboxamide.

22. Composé selon la revendication 1, qui est le sel disodique de l'acide [3S(Z)]-2-[[[1-(2-amino-4-thiazolyl)-2-[[1-[[(aminosulfonyl)amino]carbonyl]-2-oxo-3-azétidinyl]amino]-2-oxo-éthylidène]amino]oxy]-2-méthylpropanoïque.

23. Composé selon la revendication 1, qui est le chlorhydrate du (S)-N-(méthylsulfonyl)-2-oxo-3-amino-1-azétidinecarboxamide.

24. Composé selon la revendication 9, qui est le sel interne du (S)-3-amino-N-(méthylsulfonyl)-2-oxo-1-azétidinecarboxamide.

25. Composé selon la revendication 9, qui est le sel d'acide trifluoracétique du [3S-[3α,4β]]-3-amino-4-méthyl-N-(méthylsulfonyl)-2-oxo-1-azétidinecarboxamide.

26. Composé selon la revendication 9, qui est le sel d'acide trifluoracétique du (S)-3-amino-N-(méthylsulfonyl)-2-oxo-1-azétidinecarbothioamide.

27. Composé selon la revendication 9, qui est le sel interne du (S)-3-amino-N-(aminosulfonyl)-2-oxo-1-azétidinecarboxamide.

28. Composé selon la revendication 9, qui est le sel interne du (S)-3-amino-N-[(méthylamino)sulfonyl]-2-oxo-1-azétidinecarboxamide.

29. Composé selon la revendication 9, qui est le (S)-3-amino-2-oxo-N-[[(4-pyridyl) amino]sulfonyl]-1-azétidinecarboxamide.

30. Composé selon la revendication 9, qui est le sel de triéthylammonium du (S)-1-[[[(4-méthylphényl)-sulfonyl]amino]carbonyl]-2-oxo-3-aminoazétidine.

31. Composé selon la revendication 1, de formule (IV)

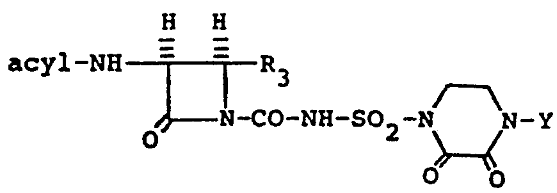

(IV)

dans laquelle Y peut être un atome d'hydrogène ou un radical alkyle éventuellement substitué, phényle éventuellement substitué, phénylalkyle ou (phényl substitué)alkyle, les définitions qui précédent étant précisées comme dans la revendication 1.

32. Composé selon la revendication 1, de formule (V)

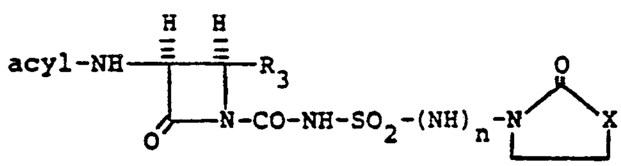

(V)

dans laquelle n peut être égal à zéro ou à un et X peut être un groupement méthylène ou un atome d'oxygène ou d'azote qui peut être substitué par un radical alkyle éventuellement substitué, phényle éventuellement substitué phénylalkyle, (phényl substitué)alkyle, amine, alkylamine, dialkylamine, alkylidèneamine, aralkylidèneamine, alkylsulfonyle, carboxy ou carboxyalkyle.

33. Composé selon la revendication 1, de formule (VI)

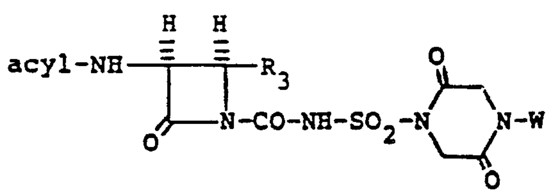

(VI)

dans laquelle W peut être un atome d'hydrogène ou un radical alkyle éventuellement substitué, phényle éventuellement substitué, phénylalkyle ou (phényl substitué)alkyle, les définitions qui précèdent étant précisées comme dans la revendication 1.